# EUROPEAN PATENT APPLICATION

(11) **EP 4 635 957 A1**
(43) Date of publication of application: **22.10.2025**
(21) Application number: 23910695.8
(22) Date of filing: 27.12.2023
(51) Int. Cl.: C07D 487/04, C07C 269/06, C07C 271/22, C07C 271/16, C07C 271/14, C07C 303/28, C07C 309/66, C07C 313/06, C07C 381/10

(54) **PREPARATION METHOD FOR STEREOISOMER AND INTERMEDIATE THEREOF**

(30) Priority: 28.12.2022 CN 202211727790
(71) Applicant: Felicamed Biotechnology Co., Ltd., Yangjiang, Guangdong 529500 (CN)
(72) Inventor: AN, Na, Zhuhai, Guangdong 519031 (CN); LIN, Xingyu, Zhuhai, Guangdong 519031 (CN); LU, Tingting, Zhuhai, Guangdong 519031 (CN); LI, Danni, Zhuhai, Guangdong 519031 (CN); ZHANG, Xue, Zhuhai, Guangdong 519031 (CN)
(74) Representative: Keller Schneider Patentanwaltsgesellschaft mbH
(86) International application number: PCT/CN2023/142264
(87) International publication number: WO 2024/140776

(57) **Abstract**

Disclosed in the present invention are a preparation method for a stereoisomer and an intermediate thereof, and a preparation method for and the use of the intermediate, in particular the use thereof in the preparation of an active compound of a JAK inhibitor. In the preparation method, in particular, a sulfoximine-structure-substituted cyclohexylamine stereoisomer is first prepared, and same is then reacted with substituted pyrrolopyrimidine to obtain a substituted heterocyclic compound having a specific three-dimensional structure, which may be directly used as an active compound or further modified to obtain more active compounds. The method has a relatively high yield, mild reaction conditions and a low cost, the levels of quality and purity of the target product are relatively high, and the method is suitable for industrial production.

## Description

### TECHNICAL FIELD

The present application relates to the field of chemical medicine technology, and in particular to a method for preparing a stereoisomer, an intermediate of the stereoisomer, a method for preparing the intermediate and a use thereof (particularly use in the preparation of a JAK inhibitor active compound).

### BACKGROUND

Stereoisomers refer to molecules with the same atomic composition and connection method but different three-dimensional spatial arrangements. They include optical isomers and geometric isomers (also called cis-trans isomers). Optical isomers are further divided into enantiomers and diastereomers. Enantiomers have many common physical and chemical properties, such as melting point, solubility, and the same types of chemical reactions, but there are also many great differences in the physical and chemical properties between enantiomers, such as optical rotation, pharmacological effects, and toxic side effects. For example, the dextroisomer of picenadol as a new phenylpiperidine analgesic is an opioid receptor agonist, while its levorotatory isomer is an opioid receptor antagonist. The dextroisomer of thalidomide has a central sedative effect, while the levorotatory isomer has severe embryonic teratogenicity.

Single optical isomer drugs have become a hot topic in new drug research and development because of their following advantages: 1. they can shorten the new drug development cycle and reduce development costs. Developing a single optical isomer drug abroad based on an existing racemic drug only requires about $3 million in funding and a four-year development cycle, while creating a completely new drug requires about $230 million in costs and 10-12 years; 2. they can enhance drug efficacy or reduce toxic and adverse reactions. For example, the β-receptor blocking activity of (S)-propranolol is 98 times stronger than that of its (R)-isomer.

The preparation process of single optical isomers mainly includes asymmetric synthesis and racemic separation. The former requires the use of an asymmetric catalyst, but effective asymmetric catalysts are difficult to obtain and difficult to synthesize. The latter has certain losses during the separation process and has a lower yield. For example, CN111499641A discloses a JAK inhibitor and a preparation method thereof, which mainly obtains each single optical isomer by final separation of the racemate, and the separation loss is large and the yield (only about 20-30% at most) is low, which is not suitable for industrial scale-up production.

### SUMMARY

To overcome the deficiencies of the prior art, the present application provides a method for preparing a stereoisomer, and an intermediate of the stereoisomer, a method for preparing the intermediate and uses thereof, in particular, uses thereof in the preparation of JAK inhibitor active compounds (e.g., the compounds described in CN111499641A).

In a first aspect of the present application, provided is a method for preparing a stereoisomer, wherein the method comprises the following steps:
(1) mixing compound RM01 with solvent 1, catalyst 1, a base and compound RM02 to react to obtain compound RM03, 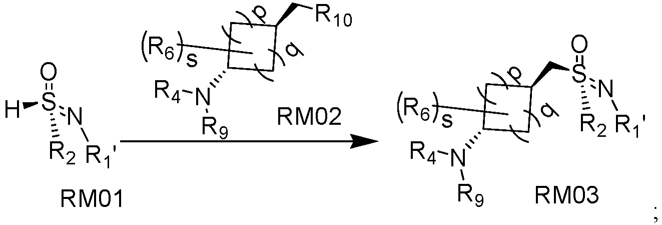
(2) deprotecting the amino group of compound RM03 to obtain compound RM04, 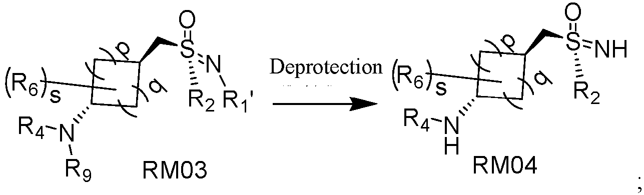 and
(3) mixing compound RM04 with solvent 3, a base, and compound RM05 to react to obtain compound RM06, 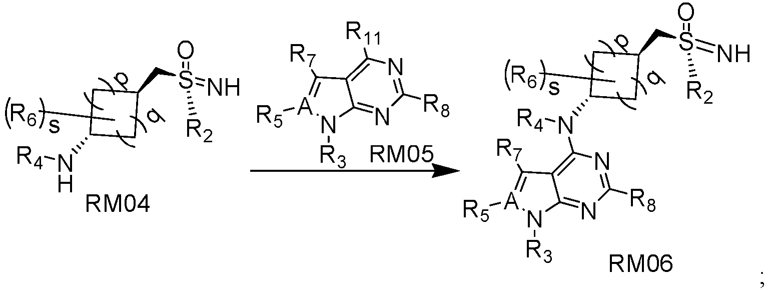
   wherein A is selected from C or N; when A is N, R₅ is absent, and when A is C, R₅ is selected from the group consisting of: H, halogen, hydroxyl, cyano, substituted or unsubstituted alkyl, substituted or unsubstituted haloalkyl, substituted or unsubstituted alkoxy, substituted or unsubstituted hydroxyalkyl, substituted or unsubstituted cycloalkyl, substituted or unsubstituted heterocycloalkyl, substituted or unsubstituted amino, substituted or unsubstituted sulfonic acid group, and substituted or unsubstituted sulfonyl;
   R₁' is an amino protecting group;
   R₂ is selected from the group consisting of: halogen, -CN, -OCH₂F, -OCHF₂, -OCF₃, C₁₋₁₀ alkyl, C₃₋₁₀ cycloalkyl, -OC₀₋₁₀ alkyl, -N(C₀₋₁₀ alkyl)(C₀₋₁₀ alkyl), -O heterocycloalkyl, -N heterocycloalkyl, -S heterocycloalkyl, C₆₋₁₈ aryl, -N heterocyclic aryl, -S heterocyclic aryl or -O heterocyclic aryl, cycloalkylalkyl, aralkyl and heterocyclylalkyl, wherein the H on the carbon or nitrogen atom may be substituted by a group selected from: deuteration, hydroxyl, halogen, -CN, -OCH₂F, -OCHF₂, -OCF₃, C₁₋₆ straight chain alkyl, -N(C₀₋₁₀ alkyl)(C₀₋₁₀ alkyl), -OC₀₋₁₀ alkyl, C₃₋₁₀ cycloalkyl, -O heterocycloalkyl, -N heterocycloalkyl, -S heterocycloalkyl, C₆₋₁₈ aryl, -N heterocyclic aryl, -O heterocyclic aryl or -S heterocyclic aryl; and wherein the H on the C₆₋₁₈ aryl or heterocyclic aryl may be substituted by any one or more groups selected from: halogen, C₁₋₄ straight chain alkyl, -N(C₀₋₁₀ alkyl)SO₂(C₀₋₁₀ alkyl), -CON(C₀₋₁₀ alkyl)(C₀₋₁₀ alkyl), -N(C₀₋₁₀ alkyl)CO(C₀₋₁₀ alkyl), -N(C₀₋₁₀ alkyl)COO(C₀₋₁₀ alkyl), -OCON(C₀₋₁₀ alkyl)(C₀₋₁₀ alkyl) -alkyl), -CN, -OCH₂F, -OCHF₂, -OCF₃, -N(C₀₋₁₀ alkyl)(C₀₋₁₀ alkyl), -OC₀₋₁₀ alkyl, -N heterocyclic aryl, -O heterocyclic aryl or -S heterocyclic aryl; or the adjacent carbon atoms on the C₆₋₁₈ aryl or heterocyclic aryl form a C₃₋₈ cycloalkyl, -O heterocycloalkyl, -N heterocycloalkyl, -S heterocycloalkyl, -N heterocyclic aryl, -O heterocyclic aryl, or -S heterocyclic aryl;
   R₃ is an amino protecting group;
   R₄ is selected from the group consisting of: H, C₁₋₁₀ straight chain/branched alkyl, C₁₋₁₀ straight chain/branched alkenyl, C₁₋₁₀ straight chain/branched alkynyl, C₆₋₁₈ aryl, C₆₋₁₈ heterocyclic aryl, C₃₋₁₀ cycloalkyl, -OC₀₋₁₀ alkyl and -O heterocycloalkyl, wherein the H on the carbon atom may be substituted by a group selected from: deuteration, hydroxyl, halogen, -CN, -OCH₂F, -OCHF₂, -OCF₃, C₁₋₁₀ straight chain/branched alkyl, -N(C₀₋₁₀ alkyl)(C₀₋₁₀ alkyl), -OC₀₋₁₀ alkyl, C₃₋₁₀ cycloalkyl, -O heterocycloalkyl, -N heterocycloalkyl, -S heterocycloalkyl, C₆₋₁₈ aryl, -N heterocyclic aryl, -O heterocyclic aryl or -S heterocyclic aryl; and wherein the alkyl moiety of the group is substituted by any one or more groups selected from: -SO₂, -SO₂N(C₀₋₁₀ alkyl)(C₀₋₁₀ alkyl), -N(C₀₋₁₀ alkyl)SO₂(C₀₋₁₀ alkyl), -CON(C₀₋₁₀ alkyl)(C₀₋₁₀ alkyl), -N(C₀₋₁₀ alkyl)CO(C₀₋₁₀ alkyl), -N(C₀₋₁₀ alkyl)COO(C₀₋₁₀ alkyl), -OCON(C₀₋₁₀ alkyl)(C₀₋₁₀ alkyl), halogen, -CN, -OCH₂F, -OCHF₂, -OCF₃, -N(C₀₋₁₀ alkyl)(C₀₋₁₀ alkyl), -OC₀₋₁₀ alkyl, C₆₋₁₈ aryl, -N heterocyclic aryl, -O heterocyclic aryl or -S heterocyclic aryl;
   R₆ is a substituent group for H on one or more carbon atoms on a cycloalkyl group, each R₆ is independently selected from the group consisting of: C₁₋₆ alkyl and C₃₋₆ cycloalkyl, s is an integer from 0-8 (e.g., 0, 1, 2, 3, 4, or 5);
   p is any integer from 0-4, q is any integer from 0-4, and p and q are not 0 at the same time;
   R₇ and R₈ are independently selected from the group consisting of: H, halogen, hydroxyl, cyano, substituted or unsubstituted alkyl, substituted or unsubstituted alkenyl, substituted or unsubstituted alkynyl, substituted or unsubstituted haloalkyl, substituted or unsubstituted alkoxy, substituted or unsubstituted hydroxyalkyl, substituted or unsubstituted cycloalkyl, substituted or unsubstituted non-heterocyclic aryl, substituted or unsubstituted heterocyclic aryl, substituted or unsubstituted heterocycloalkyl, substituted or unsubstituted amino, substituted or unsubstituted sulfonic acid group, and substituted or unsubstituted sulfonyl;
   R₉ is an amino protecting group; and
   R₁₀ and R₁₁ are independently selected from halogen.

In one embodiment of the present application, the preparation method further comprises the step of:
(4) deprotecting the amino group of compound RM06 to obtain compound I,

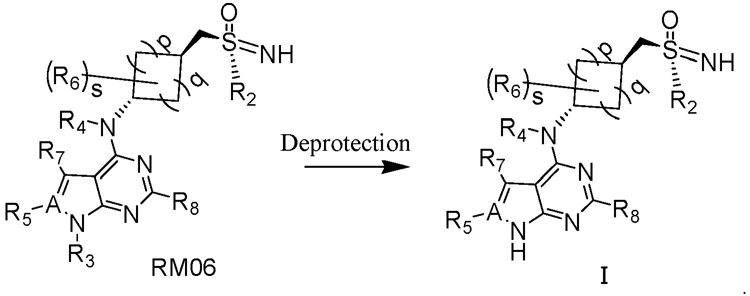

In another embodiment of the present application, the preparation method further comprises the step of:(4') reacting compound RM06 with a modifying agent to obtain compound RM07, 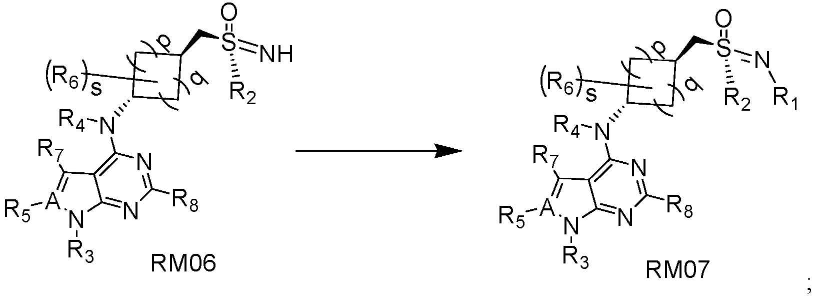 further, the preparation method also comprises the step of:
(5') deprotecting the amino group of compound RM07 to obtain compound II, 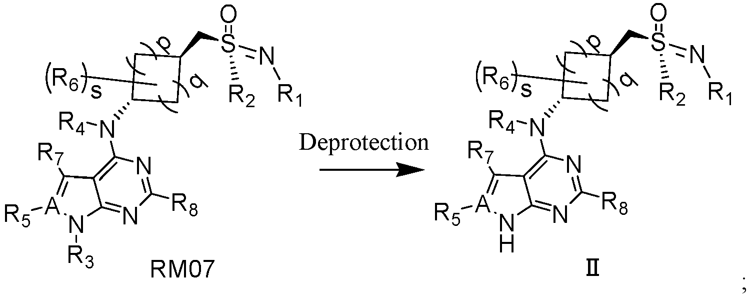
wherein
R₁ is selected from the group consisting of: halogen, -CN, -OCH₂F, -OCHF₂, -OCF₃, C₁₋₁₀ alkyl, C₃₋₁₀ cycloalkyl, -OC₀₋₁₀ alkyl, -N(C₀₋₁₀ alkyl)(C₀₋₁₀ alkyl), -O heterocycloalkyl, -N heterocycloalkyl, -S heterocycloalkyl, C₆₋₁₈ aryl, -N heterocyclic aryl, -S heterocyclic aryl or -O heterocyclic aryl, cycloalkylalkyl, aralkyl and heterocyclylalkyl, wherein the H on the carbon or nitrogen atom may be substituted by a group selected from: deuteration, hydroxyl, halogen, -CN, -OCH₂F, -OCHF₂, -OCF₃, C₁₋₆ straight chain alkyl, -N(C₀₋₁₀ alkyl)(C₀₋₁₀ alkyl), -OC₀₋₁₀ alkyl, C₃₋₁₀ cycloalkyl, -O heterocycloalkyl, -N heterocycloalkyl, -S heterocycloalkyl, C₆₋₁₈ aryl, -N heterocyclic aryl, -O heterocyclic aryl or -S heterocyclic aryl; and wherein the H on the C₆₋₁₈ aryl or heterocyclic aryl may be substituted by any one or more groups selected from: halogen, C₁₋₄ straight chain alkyl, -N(C₀₋₁₀ alkyl)SO₂(C₀₋₁₀ alkyl), -CON(C₀₋₁₀ alkyl)(C₀₋₁₀ alkyl), -N(C₀₋₁₀ alkyl)CO(C₀₋₁₀ alkyl), -N(C₀₋₁₀ alkyl)COO(C₀₋₁₀ alkyl), -OCON(C₀₋₁₀ alkyl)(C₀₋₁₀ alkyl), -CN, -OCH₂F, -OCHF₂, -OCF₃, -N(C₀₋₁₀ alkyl)(C₀₋₁₀ alkyl), -OC₀₋₁₀ alkyl, -N heterocyclic aryl, -O heterocyclic aryl or -S heterocyclic aryl; or the adjacent carbon atoms on the C₆₋₁₈ aryl or heterocyclic aryl form a C₃₋₈ cycloalkyl, -O heterocycloalkyl, -N heterocycloalkyl, -S heterocycloalkyl, -N heterocyclic aryl, -O heterocyclic aryl or -S heterocyclic aryl.

Particularly, in the above compound structures, R₁ may be selected from the group consisting of: C₁₋₆ alkyl, C₁₋₆ haloalkyl, C₃₋₆ cycloalkyl, C₃₋₆ halocycloalkyl, C₃₋₈ cycloalkyloxy, C₄₋₈ cycloalkylalkyl, C₂₋₆ alkoxyalkyl, C₆₋₁₀ aryl (e.g., phenyl), 4-10 membered heterocyclyl, C₆₋₁₀ aralkyl (e.g., benzyl, phenethyl), heterocyclylalkyl; optionally, the H on the carbon or nitrogen atom may be substituted by a group selected from the group consisting of: deuteration, hydroxyl, halogen, -CN, -CH₂-CN, -OCH₂F, -OCHF₂ and -OCF₃; more particularly, R₁ may be selected from the group consisting of: C₁₋₆ alkyl, C₁₋₆ haloalkyl, C₃₋₆ cycloalkyl, C₄₋₈ cycloalkylalkyl, and C₂₋₆ alkoxyalkyl.

In some embodiments of the present application, R₁ is selected from the group consisting of: -CH₃, -CH₂CH₃, -CH₂CH₂CH₃,

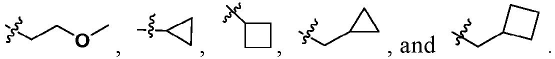

Particularly, R₂ may be selected from: C₁₋₆ alkyl, C₁₋₆ haloalkyl, C₃₋₆ cycloalkyl, C₃₋₆ halocycloalkyl, C₃₋₈ cycloalkyloxy, C₄₋₈ cycloalkylalkyl, C₂₋₆ alkoxyalkyl, C₆₋₁₀ aryl (e.g., phenyl), 4-10 membered heterocyclyl, C₆₋₁₀ aralkyl (e.g., benzyl, phenethyl), heterocyclylalkyl; optionally, wherein the H on the carbon or nitrogen atom may be substituted by a group selected from the group consisting of: deuteration, hydroxyl, halogen, -CN, -CH₂-CN, -OCH₂F, -OCHF₂, and -OCF₃, more particularly, R₂ may be selected from the group consisting of: C₁₋₆ alkyl, C₁₋₆ haloalkyl, C₃₋₆ cycloalkyl, C₄₋₈ cycloalkylalkyl, C₂₋₆ alkoxyalkyl, and C₆₋₁₀ aralkyl (e.g., benzyl, phenethyl); optionally, wherein the H on the carbon or nitrogen atom may be substituted by a group selected from the group consisting of: hydroxyl, halogen, -CN, -CH₂-CN, -OCH₂F, -OCHF₂, and -OCF₃.

In some embodiments of the present application, R₂ is selected from the group consisting of: -CH₃, -CH₂CH₃, -CH₂CH₂CH₃, ,

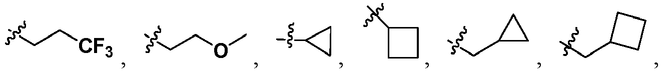

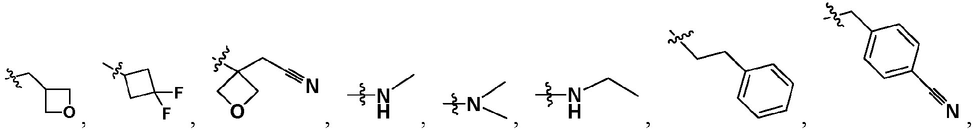

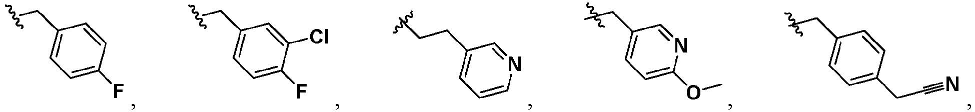

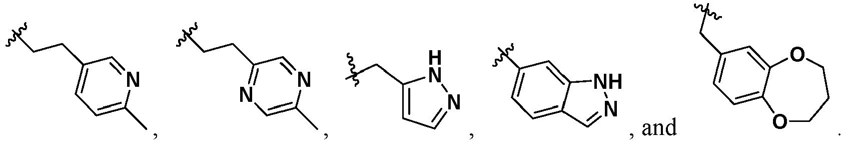

Particularly, R₄ may be selected from C₁₋₆ straight chain alkyl, and the H on the carbon atom may be substituted by a group selected from the group consisting of: deuteration, hydroxyl, -CN, -OCH₂F, -OCHF₂, -OCF₃, C₁₋₃ straight chain alkyl, and C₃₋₆ cycloalkyl.

**In** some preferred embodiments of the present application, R₄ is -CH₃.

**In** some embodiments of the present application, A is C.

Particularly, R₅ may be selected from the group consisting of: H, C₁₋₃ alkyl, and -OC₀₋₂ alkyl.

**In** some embodiments of the present application, the R₅ is selected from the group consisting of: H and -CH₃.

**In** some other embodiments of the present application, A is N.

Particularly, R₆ may be selected from C₁₋₃ alkyl, such as -CH₃, or -CH₂CH₃.

In some embodiments of the present application, s is 0.

In some embodiments of the present application, the 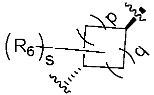 moiety is C₄₋₁₀ cycloalkyl, such as

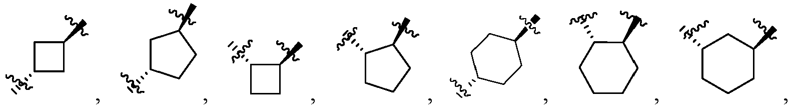

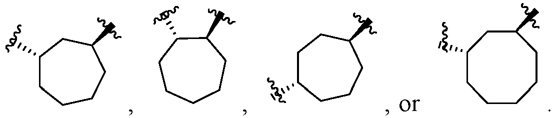

Preferably, 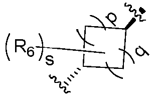 moiety is

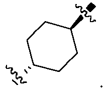

Particularly, R₇ and R₈ may be independently selected from the group consisting of: H, C₁₋₃ alkyl, and -OC₀₋₂ alkyl.

More particularly, R₇ and R₈ may be independently selected from: H and -CH₃.

In some embodiments of the present application, R₇ and R₈ are both H.

In some embodiments of the present application, the structure of compound RM02 is: 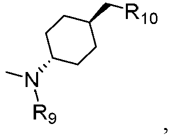 in this case, the structures of compounds RM03 and RM04 are: 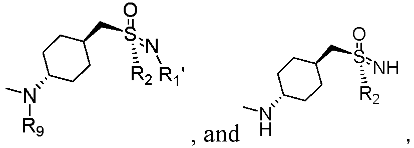 respecitively; the reaction schemes of steps (1) and (2) are as follows:

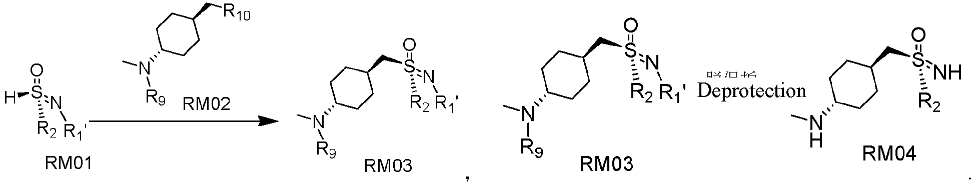

In some embodiments of the present application, the structure of compound RM05 is:

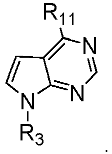

In some embodiments of the present application, the structure of compound RM06 is: 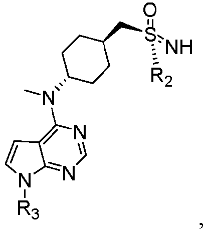 in this case, the structures of compounds I, RM07, and II successively are: 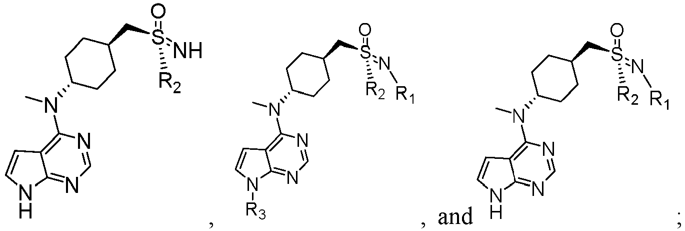 the reaction schemes of steps (3) and (4) are as follows:

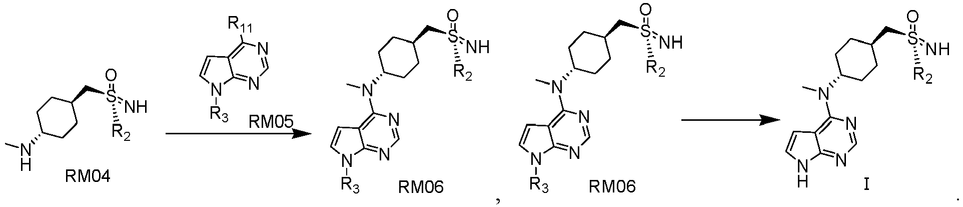

Particularly, the amino protecting group in the present application may be any suitable group, for example, an alkoxycarbonyl amino protecting group, such as Cbz (benzyloxycarbonyl), Boc (tert-butyloxycarbonyl), Fmoc (fluorenylmethyloxycarbonyl), Allo (allyloxycarbonyl), Teoc (trimethylsilylethoxycarbonyl), methoxycarbonyl, ethoxycarbonyl, etc.; acyl groups, such as Pht (phthaloyl), Ts (p-toluenesulfonyl), Ms (methylsulfonyl), Tfa (trifluoroacetyl); alkyl groups, such as Trt (trityl), Dmb (2,4-dimethoxybenzyl), Pmb (p-methoxybenzyl), Bn (benzyl).

In some embodiments of the present application, R₁' is an acyl amino protecting group, for example, R₁' is 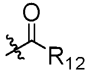 (the structure of compound RM01 is 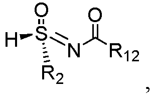 wherein R₁₂ is selected from the group consisting of: C₁₋₆ alkyl, C₁₋₆ haloalkyl and phenyl; in one embodiment of the present application, R₁' is

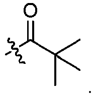

In some embodiments of the present application, R₉ is an alkoxycarbonyl amino protecting group, such as Cbz, Boc, Fmoc, Allo, Teoc, methoxycarbonyl, ethoxycarbonyl, particularly Boc.

Particularly, R₁₀ may be F, Cl, Br, or I, particularly I.

Particularly, step (1) comprises: mixing compound RM01 with solvent 1 and catalyst 1, adding a base, and then adding compound RM02 to react to obtain compound RM03.

Particularly, the solvent 1 in step (1) may be any one or more selected from the group consisting of: ethyl acetate, isopropanol, chloroform, methylethyl ketone, dioxane, pyridine, DMF, NMP, tetrahydrofuran and 2-methyltetrahydrofuran, etc., particularly dioxane.

In some embodiments of the present application, the catalyst 1 is a crown ether, such as 18-crown ether-6, or 15-crown ether-5.

Particularly, the base in step (1) may be a strong base, including inorganic strong bases, such as alkali metal hydrides (e.g., sodium hydride, potassium hydride), amino compounds (e.g., potassium amide, sodium amide); organic strong bases, such as sodium alkoxide or potassium alkoxide (e.g., sodium methoxide, sodium ethoxide, potassium ethoxide, sodium tert-butoxide, potassium tert-butoxide, etc.), organic metal lithium compounds (e.g., butyl lithium, lithium diisopropylamide, benzyl lithium), Grignard reagent, DBU, etc.; it can also be a weak base, including inorganic weak bases, such as potassium carbonate, sodium carbonate, potassium bicarbonate, sodium bicarbonate, etc., and organic weak bases, such as triethylamine, pyridine, etc.

In one embodiment of the present application, the base in step (1) is sodium hydride; in another embodiment of the present application, the base in step (1) is potassium tert-butoxide; in another embodiment of the present application, the base in step (1) is potassium carbonate.

Particularly, the base in step (1) is added in batches.

Particularly, the reaction temperature in step (1) is 20-70°C (e.g., room temperature, 30, 35, 40, 45, 50, 55, 60, 65, or 70°C), particularly 40-60°C.

As shown above, there are two protected amino groups in compound RM03, and the deprotection in step (2) may be carried out in steps or completed in one step.

In one embodiment of the present application, step (2) comprises the following reaction scheme:

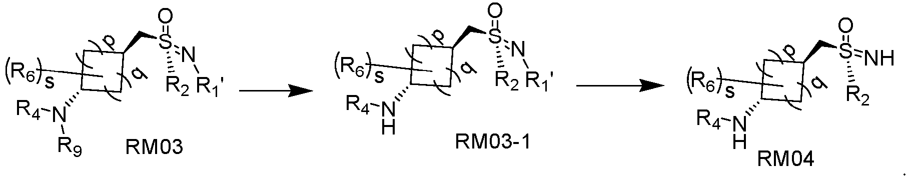

In another embodiment of the present application, step (2) comprises the following reaction scheme:

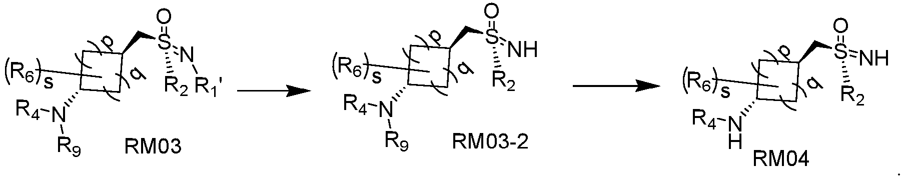

In another embodiment of the present application, the deprotection in step (2) is completed in one step.

In some embodiments of the present application, R₁' is 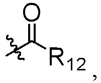 and R₉ is an alkoxycarbonyl amino protecting group.

In one embodiment of the present application, step (2) comprises: deprotecting the amino group of compound RM03 under acidic conditions to obtain compound RM04 (the deprotection of two amino groups is completed in one step). Particularly, step (2) comprises: adding acid to a reaction vessel, then adding compound RM03 to react at 50-65°C (e.g., 50, 55, 60, or 65°C, particularly 55-60°C).

Particularly, the acid is an inorganic acid, such as hydrochloric acid, sulfuric acid, nitric acid, phosphoric acid, hydrobromic acid, etc.

In another embodiment of the present application, step (2) comprises:
(2-1) mixing compound RM03 with solvent 2-1 and a reducing agent to react to obtain compound RM03-2;
(2-2) mixing compound RM03-2, solvent 2-2 and an acid to react to obtain compound RM04.

Particularly, the solvent 2-1 in step (2-1) may be any one or more selected from the group consisting of: butyl acetate, propanol, methylisobutyl ketone, tetrahydrofuran, ethyl acetate, isopropanol and chloroform, particularly tetrahydrofuran.

Particularly, the reducing agent in step (2-1) may be lithium aluminum tetrahydride, sodium borohydride, or potassium borohydride, etc., particularly lithium aluminum tetrahydride.

Particularly, the reaction temperature in step (2-1) is 5-15°C, for example 10°C.

Particularly, the solvent 2-2 in step (2-2) may be any one or more selected from the group consisting of: ethyl acetate, chloroform, dichloromethane and ether, etc., particularly dichloromethane.

Particularly, the acid in step (2-2) is a strong organic acid, such as trifluoroacetic acid or hydrochloric acid.

Particularly, the reaction temperature in step (2-2) is 20-30°C, such as room temperature.

In one embodiment of the present application, compound RM01 is 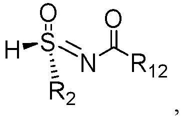 which may be prepared by a method comprising the following steps:
(a) mixing compound RM01-1 with solvent a, adding a base, and then adding compound 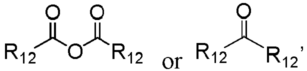 to react to obtain RM01-2;
(b) mixing compound RM01-2 with solvent b and catalyst b, adding a base, and then adding compounds R₂-R₁₄ to react to obtain compound RM01-3; and
(c) removing the R₁₃ group from compound RM01-3 to obtain compound RM01; 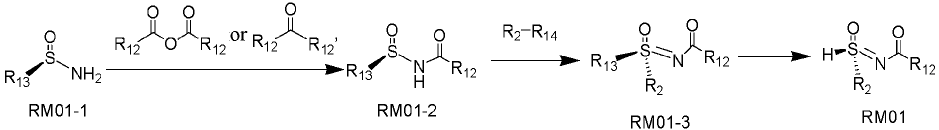 wherein
   R₁₂' is a halogen (e.g., Cl);
   R₁₃ is C₁₋₁₀ alkyl, C₃₋₁₀ cycloalkyl, or C₆₋₁₈ aryl (e.g., phenyl); and
   R₁₄ is a leaving group.

In some embodiments of the present application, R₁₃ is tert-butyl.

Particularly, R₁₄ may be any suitable leaving group, including but not limited to: halogen (e.g., F, Cl, Br, or I), alkanesulfonyloxy (e.g., 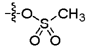 (-OMs), 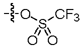 (-OTf)), arylsulfonyloxy (e.g., 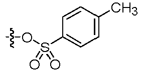 (-OTs), or 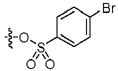 (-OBs)), etc.

Particularly, the solvent in step (a) may be any one or more selected from the group consisting of: acetone, dioxane, tetrahydrofuran, tert-butanol, and methylethyl alcohol, etc.

Particularly, the base in step (a) may be a strong base, including inorganic strong bases, such as alkali metal hydrides (e.g., sodium hydride, potassium hydride), amino compounds (e.g., potassium amide, sodium amide); organic strong bases, such as sodium alkoxide or potassium alkoxide (e.g., sodium methoxide, sodium ethoxide, potassium ethoxide, sodium tert-butoxide, potassium tert-butoxide, etc.), organic metal lithium compounds (e.g., butyl lithium, lithium diisopropylamide, benzyl lithium), Grignard reagent, DBU, etc.; it can also be a weak base, including inorganic weak bases, such as potassium carbonate, sodium carbonate, potassium bicarbonate, sodium bicarbonate, etc., and organic weak bases, such as triethylamine, pyridine, etc.

In one embodiment of the present application, the base in step (a) is sodium hydride, and the solvent a is tetrahydrofuran; in another embodiment of the present application, the base in step (a) is potassium tert-butoxide, and the solvent a is tert-butanol. In another embodiment of the present application, the base is potassium carbonate.

Particularly, the base in step (a) may be added in batches.

Particularly, the compound 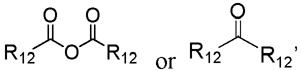 in step (a) may be added in batches or dropped.

Particularly, the reaction temperature in step (a) is 20-40°C (e.g., 20, 25, 30, 35, or 40°C).

Particularly, the solvent b in step (b) may be any one or more selected from the group consisting of: ethyl acetate, isopropanol, chloroform, methylethyl ketone, dioxane, pyridine and tetrahydrofuran, etc., particularly dioxane.

In some embodiments of the present application, the catalyst b is a crown ether, such as 18-crown ether-6, or 15-crown ether-5.

Particularly, the base in step (b) may be a strong base, including inorganic strong bases, such as alkali metal hydrides (e.g., sodium hydride, potassium hydride), amino compounds (e.g., potassium amide, sodium amide); and organic strong bases, such as sodium alkoxide or potassium alkoxide (e.g., sodium methoxide, sodium ethoxide, potassium ethoxide, sodium tert-butoxide, potassium tert-butoxide, etc.), organic metal lithium compounds (e.g., butyl lithium, lithium diisopropylamide, benzyl lithium), Grignard reagent, DBU, etc.; it can also be a weak base, including inorganic weak bases, such as potassium carbonate, sodium carbonate, potassium bicarbonate, sodium bicarbonate, etc., and organic weak bases, such as triethylamine, pyridine, etc.; in particular, the base in step (b) is a strong base.

In one embodiment of the present application, the base in step (b) is sodium hydride; in another embodiment of the present application, the base in step (b) is potassium tert-butoxide.

Particularly, the base in step (b) is added in batches.

Particularly, the reaction temperature in step (b) is 35-50°C (e.g., 35, 40, 45, or 50°C), particularly 40-45°C.

Particularly, the elimination reaction in step (c) may be carried out in the presence of a strong organic acid (e.g., trifluoroacetic acid).

Particularly, in step (3), R₁₁ may be F, Cl, Br or I, particularly Cl.

Particularly, the solvent 3 in step (3) may be any one or more selected from the group consisting of: dimethylformamide, methanol, acetic acid, acetonitrile, ethyl acetate, isopropanol, acetone and tetrahydrofuran, etc.

In one embodiment of the present application, the solvent 3 is dimethylformamide; in another embodiment of the present application, the solvent 3 is tetrahydrofuran.

Particularly, the base in step (3) is a weak base, including inorganic weak bases, such as potassium carbonate, sodium carbonate, potassium bicarbonate, sodium bicarbonate, etc., and organic weak bases, such as triethylamine, pyridine, etc., particularly inorganic weak bases.

In some embodiments of the present application, the base is potassium carbonate.

Particularly, the reaction in step (3) is carried out under heating conditions, and the reaction temperature may be 50-120°C (e.g., 50, 55, 60, 65, 70, 75, 80, 90, 100, 110, or 115°C), particularly 55-70°C.

In a preferred embodiment of the present application, steps (2) and (3) may be carried out in a one-pot process, that is, the step of preparing compound RM06 from compound RM03 may comprise:
(A) mixing compound RM03 with an acid to react at 50-65°C (e.g., 50, 55, 60, or 65°C, particularly 55-60°C);
(B) adjusting the pH to weak alkalinity; and
(C) adding a base, solvent 3 and compound RM05 to react at 55-70°C (e.g., 55, 60, 65, or 70°C, particularly 60-65°C).

Particularly, the acid in step (A) is an inorganic acid, such as hydrochloric acid, sulfuric acid, nitric acid, phosphoric acid, hydrobromic acid, etc.

In a preferred embodiment, step (A) comprises: mixing compound RM03 with an acid, stirring (for e.g., 1-2 hours) at 20-30°C (e.g., 20, 25, or 30°C, particularly room temperature), and then stirring (for e.g., 4, 5, 6, 8, or 10 hours) at 50-65°C (e.g., 50, 55, 60, or 65°C, particularly 55-60°C).

Particularly, step (B) further comprises a cooling step; more particularly, in the cooling step the temperature is decreased to 20-30°C (particularly room temperature).

Particularly, the weak alkalinity in step (B) may be pH 7-8, which may be achieved by adding a weak base (e.g., an inorganic weak base, such as potassium carbonate, sodium carbonate, potassium bicarbonate, sodium bicarbonate, etc.).

In a preferred embodiment, step (B) comprises: adjusting the pH to weak alkalinity, and stirring (for e.g., 10-30 minutes) at 20-30°C (e.g., 20, 25, or 30°C, particularly room temperature).

Particularly, the base in step (C) is a weak base, including inorganic weak bases, such as potassium carbonate, sodium carbonate, potassium bicarbonate, sodium bicarbonate, etc., and organic weak bases, such as triethylamine, pyridine, etc., particularly inorganic weak bases.

In a preferred embodiment, step (C) comprises: adding a base, solvent 3, and compound RM05 to stir (for e.g., 10, 12, 14, 16, 18, or 20 hours) at 55-70°C (e.g., 55, 60, 65, or 70°C, particularly 60-65°C).

In some embodiments of the present application, R₃ is an acyl amino protecting group, such as Pht, Ts, Ms or Tfa, particularly Ts.

Particularly, step (4) may comprise: mixing compound RM06 with solvent 4 and an alkaline reagent or a base to react to obtain compound I.

In some embodiments of the present application, step (4) comprises: mixing compound RM06 with solvent 4 and TBAF, and subjecting the mixture to a reflux reaction to obtain compound I.

In other embodiments of the present application, step (4) comprises: mixing compound RM06 with solvent 4 and a base (e.g., potassium hydroxide, sodium hydroxide) to react at 50-70°C (e.g., 50, 55, 60, 65, or 70°C) to obtain compound I.

Particularly, the solvent 4 is an organic solvent, for example, an alcohol, such as tert-butanol.

In other embodiments of the present application, step (4) comprises: mixing compound RM06 with a base (e.g., potassium hydroxide, sodium hydroxide) and water to react at 50-70°C (e.g., 50, 55, 60, 65, or 70°C); particularly, step (4) further comprises: diluting the reaction product, adjusting the pH to acidic, stirring at 25-30°C, then adjusting the pH to weak alkalinity, stirring at 25-30°C, and then separating the organic layer for purification. Particularly, the modification reagent and reaction in step (4') may be selected according to the R₁ group. For example, if R₁ is methyl, the modification reagent may be polyformaldehyde; if R₁ is ethyl, the modification reagent may be triethyloxonium tetrafluoroborate; and so on.

Particularly, step (5') can refer to step (4).

In one embodiment of the present application, compound RM02 is 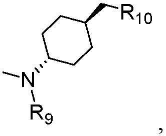 which may be prepared by the following reaction scheme: 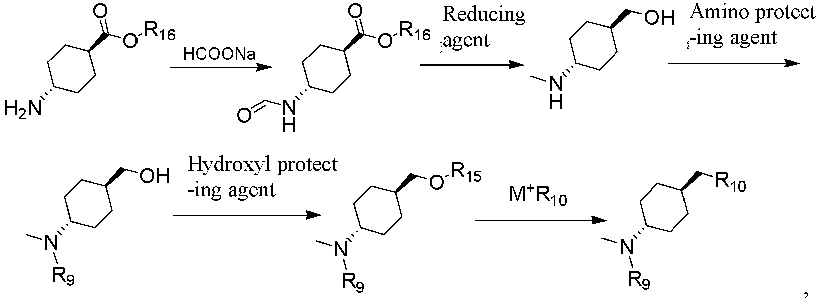 wherein
R₁₅ is a hydroxyl protecting group;
R₁₆ is H or alkyl; and
M is an alkali metal (e.g., Na).

In another embodiment of the present application, compound RM02 is 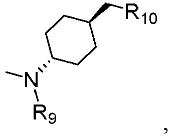 which may be prepared by the following reaction scheme: 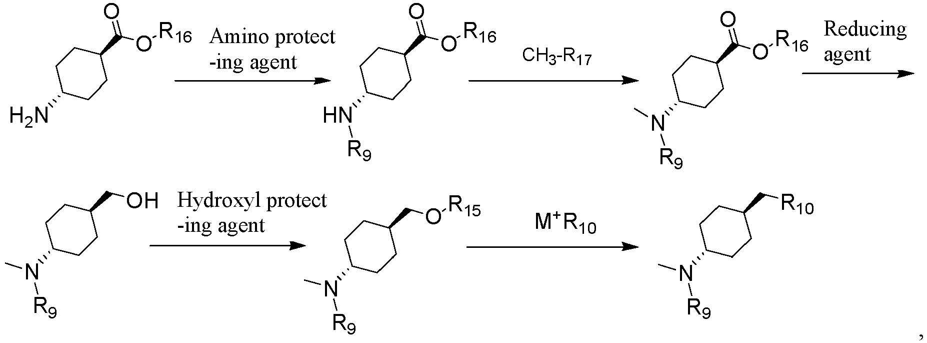 wherein
R₁₃ is a hydroxyl protecting group;
R₁₆ is H or alkyl;
R₁₇ is halogen (e.g., I); and
M is an alkali metal (e.g., Na).

Particularly, the hydroxyl protecting group in the present application may be any suitable group, for example, a silyl ether protecting group (e.g., -Si(CH₃)₃, 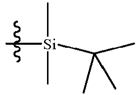 ), a benzyl ether protecting group, an alkyl ether protecting group (for example, -CH₃, -C(CH₃)₃), an alkoxymethyl ether protecting group (for example, -CH₂OCH₃), an acyl protecting group (for example, -COCH₃, -COC(CH₃)₃, Ms, Ts).

In some embodiments of the present application, R₁₅ is Ms or Ts.

In some embodiments of the present application, R₁₆ is a C₁₋₃ alkyl group, such as a methyl group.

Particularly, the reducing agent may be sodium borohydride, sodium hydride, boron trifluoride, or the like.

Particularly, each step as described above may further comprise purification and separation of the synthetic product, for example, by using any one or a combination of more commonly used separation means selected from the group consisting of: extraction, column chromatography, crystallization, liquid separation, distillation, filtration, evaporation, washing, and drying (removing water), etc., to obtain a synthetic product that is as pure as possible.

In a second aspect of the present application, provided is an intermediate compound RM01, which may be used for the preparation of sulfoximine-substituted cycloalkylamine stereoisomers (as described in the first aspect), and the intermediate compound has the following structure: 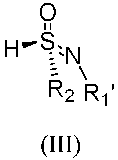 wherein
R₁' and R₂ respectively have the definitions described in the first aspect.

In some embodiments of the present application, R₁' is an acyl amino protecting group, for example, R₁' is 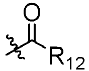 (the structure of compound RM01 is 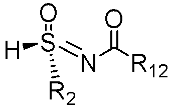 ), wherein R₁₂ is selected from the group consisting of: C₁₋₆ alkyl, C₁₋₆ haloalkyl, and phenyl; in one embodiment of the present application, R₁' is

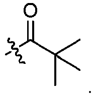

In some embodiments of the present application, the intermediate compound RM01 has the following structures:

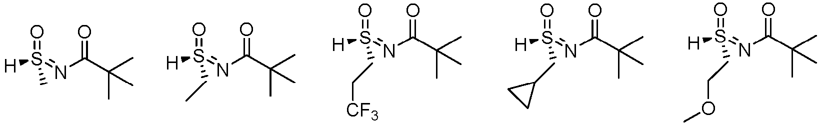

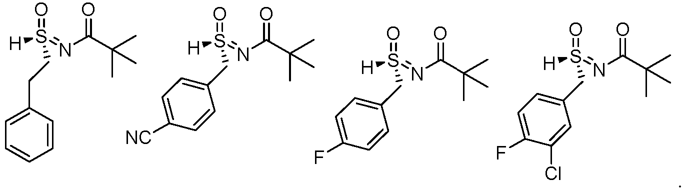

In a third aspect of the present application, provided is a method for preparing the intermediate compound RM01, which comprises the following steps:
(a) mixing compound RM01-1 with solvent a, adding a base, and then adding compound 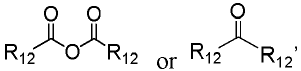 to react to obtain RM01-2;
(b) mixing compound RM01-2 with solvent b and catalyst b, adding a base, and then adding compounds R₂-R₁₄ to react to obtain compound RM01-3; and
(c) removing the R₁₃ group from compound RM01-3 to obtain compound RM01; 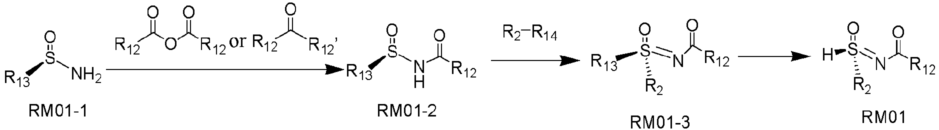 wherein
   R₁₂' is a halogen (e.g., Cl);
   R₁₃ is C₁₋₁₀ alkyl, C₃₋₁₀ cycloalkyl, or C₆₋₁₈ aryl (e.g., phenyl); and
   R₁₄ is a leaving group.

Particularly, steps (a)-(c) are as described in the first aspect of the present application.

In a fourth aspect of the present application, provided is an intermediate compound RM03, which may be used for the preparation of cycloalkylamine-substituted heterocyclic stereoisomers (as described in the first aspect), and the intermediate compound has the following structure: 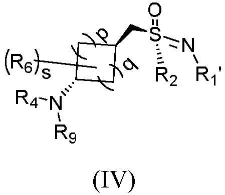 wherein
R₁', R₂, R₄, R₆, R₉, p, q, and s respectively have the definitions described in the first aspect.

In one embodiment of the present application, the structure of the intermediate compound RM03 may be:

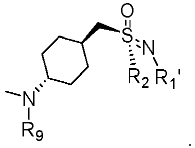

In some embodiments of the present application, R₁' is 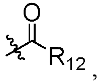 wherein R₁₂ is selected from the group consisting of: C₁₋₆ alkyl, C₁₋₆ haloalkyl, and phenyl; in one embodiment of the present application, R₁' is

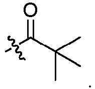

In some embodiments of the present application, the intermediate compound RM03 has the following structures:

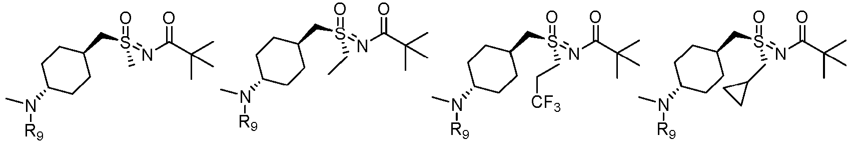

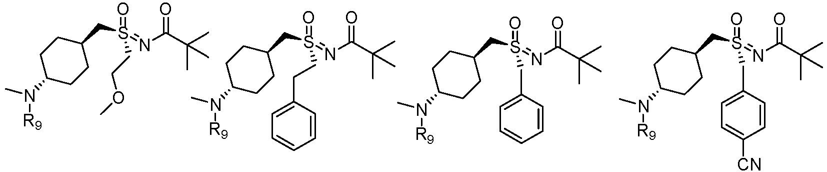

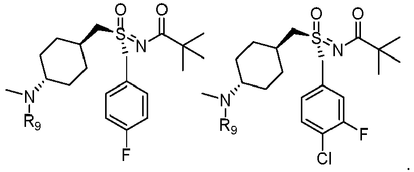

In some embodiments of the present application, R₉ is an alkoxycarbonyl amino protecting group, such as Cbz, Boc, Fmoc, Allo, Teoc, methoxycarbonyl and ethoxycarbonyl, particularly Boc.

In a fifth aspect of the present application, provided is a method for preparing the intermediate compound RM03, which comprises the following steps:
(1) mixing compound RM01 with solvent 1 and catalyst 1, adding a base, and then adding compound RM02 to react to obtain an intermediate compound RM03;
The details are as described in step (1) of the first aspect of the present application.

In a sixth aspect of the present application, provided is an intermediate compound RM06, which may be used for the preparation of cycloalkylamine-substituted heterocyclic stereoisomers (as described in the first aspect), and the intermediate compound has the following structure: 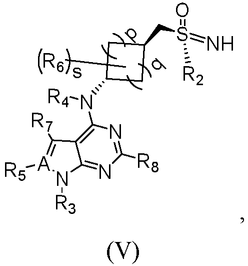 wherein
R₂, R₃, R₄, R₅, R₆, R₇, R₈, R₉, p, q, and s respectively have the definitions described in the first aspect.

In some embodiments of the present application, the structure of the intermediate compound RM06 is:

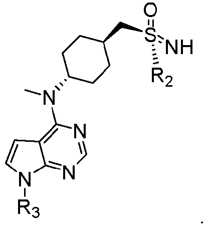

In a seventh aspect of the present application, provided is a method for preparing the intermediate compound RM06, which comprises the following steps:
(2) deprotecting the amino group of compound RM03 to obtain compound RM04; and
(3) mixing compound RM04 with solvent 3, a base, and compound RM05 to react to obtain compound RM06;

The details are as described in the first aspect of the present application for steps (2) and (3).

In a preferred embodiment of the present application, steps (2) and (3) may be carried out in a one-pot process, that is, the preparation method of the intermediate compound RM06 may comprise the following steps:
(A) mixing compound RM03 with an acid to react at 50-65°C (e.g., 50, 55, 60, or 65° C, particularly 55-60°C);
(B) adjusting the pH to weak alkalinity; and
(C) adding a base, solvent 3 and compound RM05 to react at 55-70°C (e.g., 55, 60, 65, or 70°C, particularly 60-65°C).

Particularly, the acid in step (A) is an inorganic acid, such as hydrochloric acid, sulfuric acid, nitric acid, phosphoric acid, hydrobromic acid, etc.

In a preferred embodiment, step (A) comprises: mixing compound RM03 with an acid, stirring (for e.g., 1-2 hours) at 20-30°C (e.g., 20, 25, or 30°C, particularly room temperature), and then stirring (for e.g., 4, 5, 6, 8, or 10 hours) at 50-65°C (e.g., 50, 55, 60, or 65°C, particularly 55-60°C).

Particularly, step (B) further comprises a cooling step; more particularly, in the cooling step the temperature is decreased to 20-30°C (particularly room temperature).

Particularly, the weak alkalinity in step (B) may be pH 7-8, which may be achieved by adding a weak base (e.g., an inorganic weak base, such as potassium carbonate, sodium carbonate, potassium bicarbonate, sodium bicarbonate, etc.).

In a preferred embodiment, step (B) comprises: adjusting the pH to weak alkalinity, and stirring (for e.g., 10-30 minutes) at 20-30°C (e.g., 20, 25, or 30°C, particularly room temperature).

Particularly, the base in step (C) is a weak base, including inorganic weak bases, such as potassium carbonate, sodium carbonate, potassium bicarbonate, sodium bicarbonate, etc., and organic weak bases, such as triethylamine, pyridine, etc., particularly inorganic weak bases.

In a preferred embodiment, step (C) comprises: adding a base, solvent 3, and compound RM05, and stirring (for e.g., 10, 12, 14, 16, 18, or 20 hours) at 55-70°C (e.g., 55, 60, 65, or 70°C, particularly 60-65°C).

In an eighth aspect of the present application, provided is use of the intermediate compounds RM01, RM03, RM06 in the preparation of stereoisomers (e.g., structure formulas I and II as described in the first aspect of the present application).

In a ninth aspect of the present application, provided is a stereoisomer, which has the following structure: 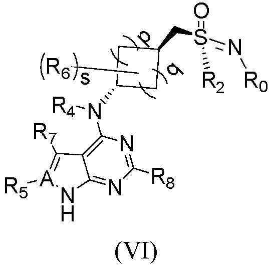
wherein R₀ is H or R₁;
R₂, R₄, R₅, R₆, R₇, R₈, p, q, s, and A respectively have the definitions described in the first aspect.

In some embodiments of the present application, the stereoisomer is 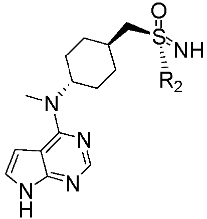 or

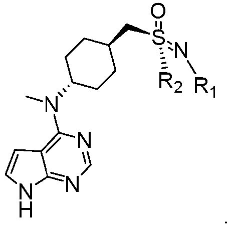

In one embodiment of the present application, the stereoisomer is

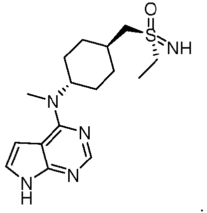

The present application provides a method for preparing a stereoisomer, and an intermediate used in the method, a method for preparing the intermediate and use thereof, in particular use thereof in the preparation of a JAK inhibitor active compound. In the preparation method, particularly, a cyclohexylamine stereoisomer substituted with a sulfoximine structure is first prepared, then reacting with a substituted pyrrolopyrimidine to obtain a substituted heterocyclic compound with a specific stereostructure, which may be directly used as an active compound or further modified to obtain more active compounds. The method has a high yield, performs at mild reaction conditions with low cost, obtains high quality and purity of the target product, and is suitable for industrial production.

### BRIEF DESCRIPTION OF THE DRAWINGS

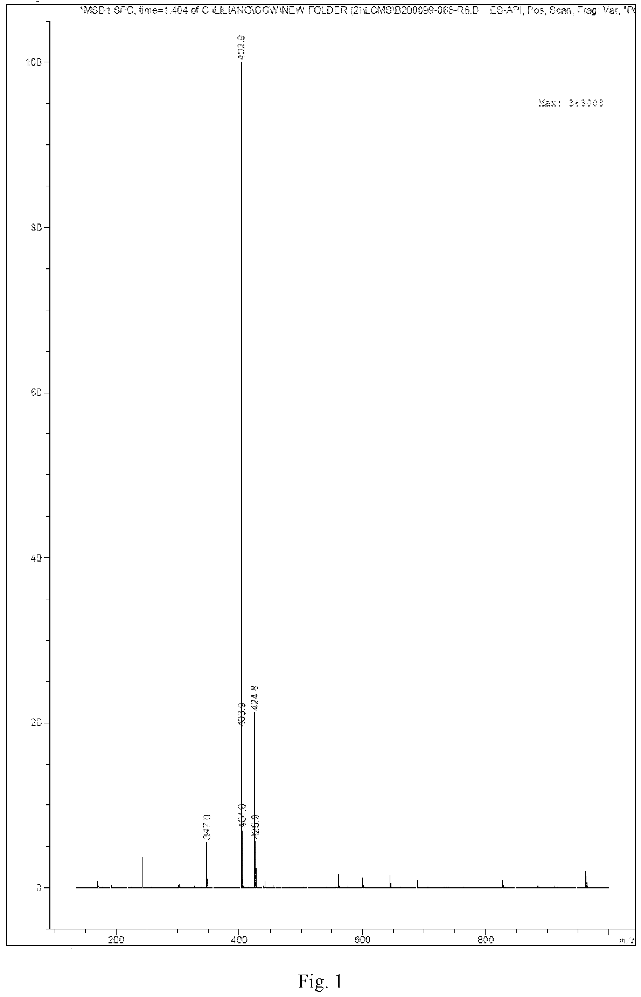

Fig. 1 shows the MS spectrum of compound 11.

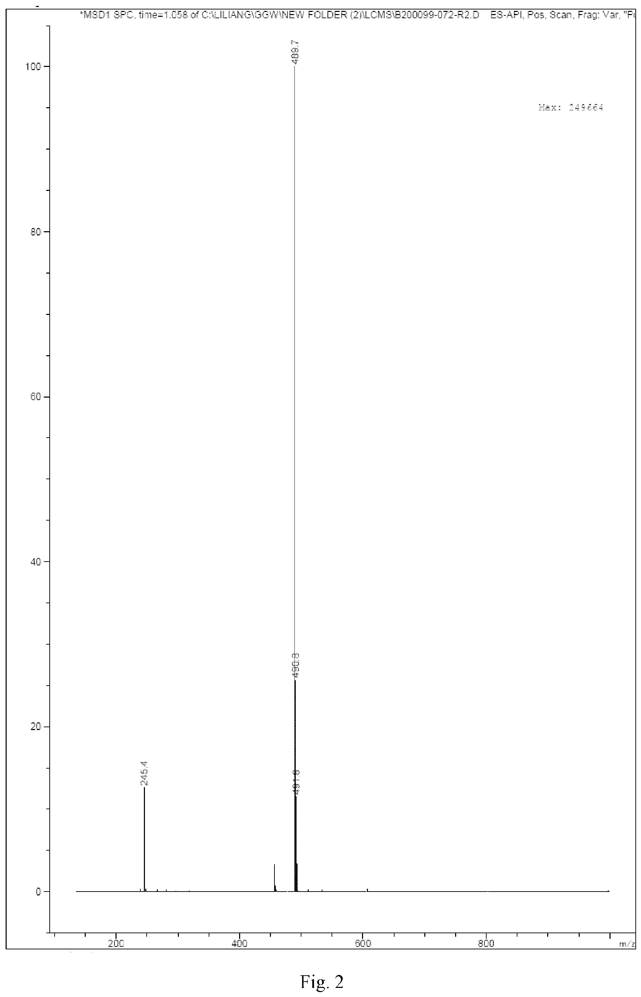

Fig. 2 shows the MS spectrum of compound 15.

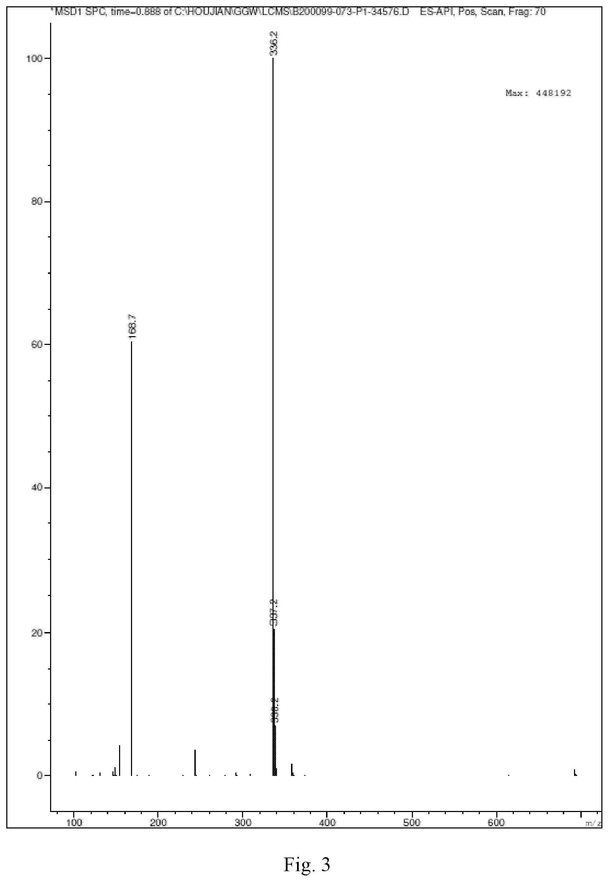

Fig. 3 shows the MS spectrum of compound T1.

### DETAILED DESCRIPTION

Unless otherwise defined, all scientific and technical terms used in the present application have the same meanings as commonly understood by a person skilled in the art to which the present application relates.

The term "alkyl" refers to a straight or branched hydrocarbon chain radical having no unsaturated bonds and it is connected to the rest of the molecule by a single bond. Typical alkyl groups comprise 1-10 (e.g., 1, 2, 3, 4, 5, 6, 7, 8, 9, or 10) carbon atoms, particularly 1-6 carbon atoms, such as methyl, ethyl, n-propyl, isopropyl, n-butyl, isobutyl, tert-butyl, n-pentyl, isopentyl, neopentyl, tert-pentyl, n-hexyl, isohexyl, etc. If an alkyl group is substituted by a cycloalkyl group, it is correspondingly a "cycloalkylalkyl" group, such as cyclopropylmethyl, cyclopropylethyl, cyclobutylmethyl, cyclopentylmethyl, cyclohexylmethyl, etc. If an alkyl group is substituted with an aryl group, it is correspondingly an "aralkyl group", such as benzyl, benzhydryl or phenethyl. If an alkyl group is substituted by a heterocyclyl group, it is correspondingly a "heterocyclylalkyl group". If an alkyl group is substituted by an alkoxy group, it is correspondingly an "alkoxyalkyl group".

The term "cycloalkyl" refers to an alicyclic hydrocarbon which may be a monocyclic, bicyclic, tricyclic, or tetracyclic ring system, which may be a fused, spirocyclic or bridged ring system. The cycloalkyl group may comprise 3-18 carbon atoms, preferably 3-10 (e.g., 3, 4, 5, 6, 7, 8, 9, or 10) carbon atoms, particularly a monocyclic group comprising 3-6 carbon atoms, such as cyclopropyl, cyclobutyl, cyclopentyl, cyclohexyl or adamantyl, etc.

The term "alkoxy" refers to a substituent formed by replacing the hydrogen in a hydroxy group with an alkyl group, such as an alkoxy group comprising 1-10 carbon atoms, for example, methoxy, ethoxy, propoxy, butoxy, etc.

The term "halogen" refers to fluorine, chlorine, bromine or iodine.

The term "haloalkyl" refers to a group formed by replacing one or more hydrogen atoms in an alkyl group with halogen atom(s) (e.g., fluorine, chlorine, bromine or iodine), for example, -CHF₂, -CH₂F, -CH₂Cl, -CF₃, -CH₂-CF₃, -CH₂CH₂-CF₃, -CH₂CH₂CH₂-CF₃.

The term "aryl" refers to a monocyclic or polycyclic free radical, including a polycyclic free radical comprising a monoaryl group and/or a fused aryl group, such as a radical comprising 1-3 monocyclic or fused rings and 6-18 (e.g., 6, 8, 10, 12, 14, 16, or 18) carbon ring atoms, such as phenyl, naphthyl, biphenyl, indenyl, etc.

The term "heterocyclyl" refers to a 3- to 18-membered non-aromatic ring group comprising 2-17 carbon atoms and 1-10 heteroatoms. The heterocyclyl may be a monocyclic, bicyclic, tricyclic, or tetracyclic ring system, which may be a fused, spirocyclic or bridged ring system. A heterocyclyl group may be partially saturated (heteroaryl) or fully saturated (heterocycloalkyl). Suitable heteroaryl groups in the compounds of the present application comprise 1, 2 or 3 heteroatoms selected from N, O or S atoms; and the heteroaryl includes, for example, coumarin (including 8-coumarin), quinolyl (including 8-quinolyl), isoquinolyl, pyridinyl, pyrazinyl, pyrazolyl, pyrimidinyl, furanyl, pyrrolyl, thienyl, thiazolyl, isothiazolyl, triazolyl, tetrazolyl, isoxazolyl, oxazolyl, imidazolyl, indolyl, isoindolyl, indazolyl, indolizinyl, phthalazinyl, pteridinyl, purinyl, oxadiazolyl, thiadiazolyl, furazanyl, pyridazinyl, triazinyl, cinnolinyl, benzimidazolyl, benzofuranyl, benzofurazanyl, benzothienyl, benzothiazolyl, benzoxazolyl, quinazolinyl, quinoxalinyl, naphthyridinyl and furopyridinyl. Suitable heterocycloalkyl groups in the compounds of the present application comprise 1, 2 or 3 heteroatoms selected from N, O or S atoms; and the heterocycloalkyl includes, for example, pyrrolidinyl, tetrahydrofuranyl, dihydrofuranyl, tetrahydrothiophenyl, tetrahydrothiopyranyl, piperidinyl, morpholinyl, thiomorpholinyl, oxathiolanyl, piperazinyl, azetidinyl, oxetanyl, thietanyl, homopiperidinyl, oxiranyl, thiiranyl, azepinyl, oxazepinyl, diazepinyl, 1,2,3,6-tetrahydropyridinyl, 2-pyrrolinyl, 3-pyrrolinyl, dihydroindolinyl, 2H-pyranyl, 4H-pyranyl, dioxanyl, 1,3-dioxolanyl, pyrazolinyl, dithianyl, dithiolanyl, dihydropyranyl, dihydrothienyl, pyrazolidinyl, imidazolinyl, imidazolidinyl, 3-azabicyclo[3.1.0]hexyl, 3-azabicyclo[4.1.0]heptyl, 3H-indolyl and quinolizinyl.

In the present application, "D" refers to deuterium; "substituted by deuterium" means replacing one or more hydrogen atoms with a corresponding number of deuterium atoms.

In the present application, there is a description of "protecting group". When a multifunctional organic compound is reacted, in order to make the reaction occur only at the desired group and avoid affecting other groups, other groups are protected before the reaction and restored after the reaction is completed. In order to protect the group, the substituent introduced on the group is called a "protecting group", and the reagent that can protect a certain group is called a protecting agent for the group. For example, (Boc)₂O may be used as a protecting agent for amino group, and the corresponding amino protecting group is Boc; for example, MsCl may be used as a protecting agent for hydroxyl group, and the corresponding hydroxyl protecting group is Ms. For a general description of protecting groups and their uses, see T. W. Greene, Protective Groups in Organic Synthesis, John Wiley & Sons, New York, 1991.

In the present application, the term "leaving group" is given its conventional meaning in the field of synthetic organic chemistry, and refers to an atom or group capable of being displaced by a nucleophile. See, for example, Smith, March Advanced Organic Chemistry, 6th Edition, (501-502).

Various publications, patents, and published patent specifications are cited in this application, the disclosures of which are incorporated herein by reference in their entireties.

The technical solution of the present application will be clearly and completely described below in conjunction with the Examples of the present application. Obviously, the described Examples are only part of the examples of the present application, rather than all the examples. Based on the Examples described in the present application, all other examples obtained by ordinary technicians in the field without making any creative work shall fall within the protection scope of the present application.

### Example 1

The synthetic scheme is as follows:

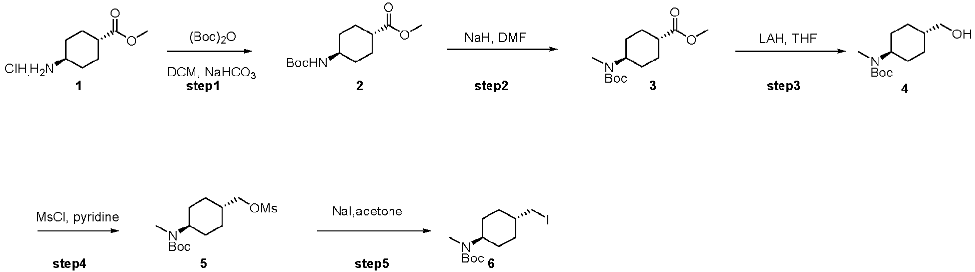

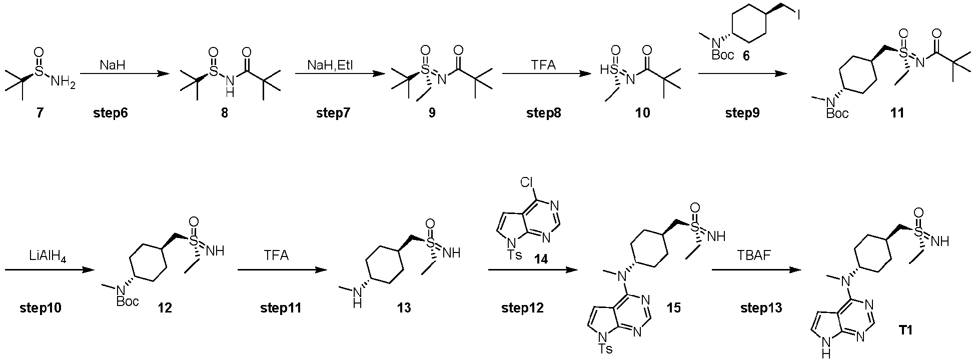

### Step 1: Synthesis of compound 2

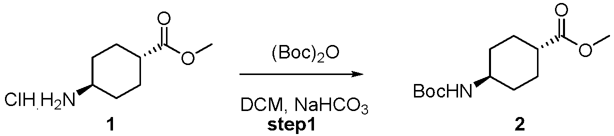

Compound 1 (500 g, 2.58 mol), dichloromethane (1 L) and purified water (1 L) were added into a 5 L three-necked flask in sequence at room temperature, and mechanical stirring was started, then adding sodium bicarbonate (400 g, 4.76 mol) and di-tert-butyl dicarbonate (650 g, 2.98 mol) in sequence. After completion of the addition, dichloromethane (1 L) and purified water (1 L) were added after stirring at room temperature for 4 hours. The liquids were separated, and the aqueous phase was extracted with dichloromethane (800 ml × 2). The organic phases were combined to wash with saturated brine (1000 ml × 1). The organic phase was dried over anhydrous sodium sulfate, filtering, and concentrating the filtrate under reduced pressure at 40°C until no fraction was distilled out. Petroleum ether (500 ml) was added to pulp at room temperature for 0.5 hours, then filtering under reduced pressure. The filter cake was collected and dried by forced air at 25°C for 16 hours to obtain 513 g of a white solid with a yield of 77%.

Ms: 280.0 [M+Na]⁺. ¹H NMR (300MHz, CDCl₃) δ 4.38 (s, 1H), 3.67 (s, 3H), 3.42 (s, 1H), 2.31-2.16 (m, 1H), 2.09-1.98 (m, 4H), 1.61-1.48 (m, 2H), 1.44 (s, 9H), 1.17-1.03 (m, 2H).

### Step 2: Synthesis of compound 3

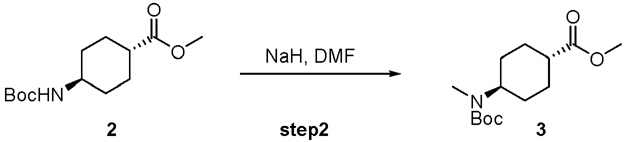

Compound 2 (513 g, 2.58 mol) and anhydrous DMF (2 L) were added into a 5 L three-necked flask at room temperature, and mechanical stirring was started, then replacing with nitrogen under reduced pressure for three times while the temperature was controlled at 10°C in an ice bath. Sodium hydride (103.8 g, 2.59 mol) was added in batches, and heat release was not obvious; after completion of the addition, the mixture was stirred for 0.5 hour while heating with warm water to return to room temperature. Iodomethane (340 g, 2.40 mol) was added dropwise, and heat release was not obvious; the addition was completed in 100 minutes, after slowly heating to 36-38°C, a violent reaction began while releasing a large amount of heat (uncontrollable), and then the mixture was cooled to room temperature to stir overnight. The reaction solution was slowly poured into 2L saturated NH₄Cl solution (the temperature was controlled below 20°C), then extracting with EA (1L×3); the organic phase was washed with saturated NaCl (500mL×3), and the organic phase was concentrated to obtain 550g of an oily substance. After wet column chromatography, elution was performed with PE/EA=10/1 to 5/1 to obtain 517g of a colorless oily substance with a yield of 95.9%.

Ms: 294.0 [M+Na]⁺. ¹H NMR (300 MHz, CDCl₃) δ 3.62 (s, 3H), 2.68 (s, 3H), 2.28-2.10 (m, 1H), 2.03-2.01 (m, 2H), 1.73-1.70 (m, 2H), 1.53-1.48 (m, 3H), 1.41-1.38 (m, 11H).

### Step 3: Synthesis of compound 4

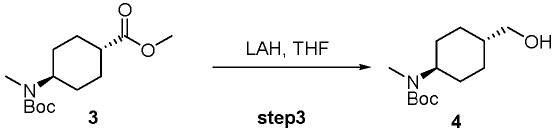

Anhydrous tetrahydrofuran (300 mL) was added into a 5L three-necked flask, and the temperature was controlled at 10-15°C in an ice bath, starting stirring under the protection of N₂, and adding lithium aluminum tetrahydride (46 g, 1.21 mol) in batches while controlling the temperature below -5°C. Then 3 (516 g, 1.90 mol) of anhydrous tetrahydrofuran solution (1.5 L) was added dropwise to react for 2.5 hours after the addition, adding 46 mL of 15% sodium hydroxide solution and 135 mL of water dropwise while controlling the temperature at 0-10°C, and filtering with suction under reduced pressure after the addition. The filter cake was rinsed with 500 mL of ethyl acetate, collecting the filtrate after filtering with suction, adding 500 ml of ethyl acetate and 1000 ml of water to the filtrate, separating the liquids, and collecting the organic phase; the aqueous phase was extracted again with EA (500 ml × 3), combining the organic phases and washing with saturated sodium chloride solution (500 ml × 3), then drying and concentrating to obtain 407 g of colorless oil with a yield of 88.1%.

Ms:266.0[M+Na]⁺. ¹H NMR(400MHz, CDCl₃)δ3.41(d,J = 6.4Hz,2H),2.69(s,3H),2.07(s,1H),1.87-1.81(m,2H),1.73-1.64(m,2H),1.43(s,10H),1.40-1.33(m,2H),1.08-0.98(m,2H).

### Step 4: Synthesis of compound 5

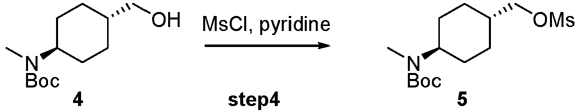

Compound 4 (407 g, 1.67 mol), anhydrous dichloromethane (1000 ml) and pyridine (304.3 g, 3.9 mol) were added into a 3L three-necked flask in sequence at room temperature, and stirring was started under nitrogen protection. Methanesulfonyl chloride (250 g, 2.18 mol) was added dropwise while the temperature was controlled at 10-15°C in an ice bath; after the addition was completed, the mixture was stirred at room temperature overnight. 500 mL of water was added to quench the reaction. The aqueous phase was extracted with dichloromethane (500 mL×3), washing with saturated NaCl solution (1000 ml×1), drying and concentrating to obtain 510.2 g of a yellow oil with a yield of 94.5%.

Ms: 343.9 [M+Na]⁺. ¹H NMR (300 MHz, CDCl₃) δ 4.02 (d, *J* = 6.4 Hz, 2H), 3.00 (s, 3H), 2.71 (s, 3H), 1.91-1.86 (m 2H), 1.71-1.63 (m 3H), 1.54 - 1.51(m, 12H), 1.18 - 1.05 (m, 2H).

### Step 5: Synthesis of compound 6

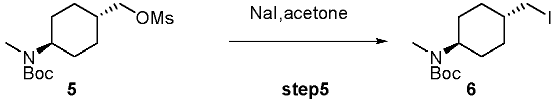

Compound 5 (510 g, 1.59 mol), acetone (3000 ml) and NaI (310 g, 2.07 mol) were added into a 10 L three-necked flask in sequence at room temperature, and stirring was started. The reaction mixture was heated under reflux overnight, cooling to room temperature, and adding water (1000 ml) to quench the reaction. The mixture was then extracted with ethyl acetate (1 L × 3), then washing with 2 L of saturated NaCl, drying and concentrating to obtain 423 g of an oily substance, which was used directly in the next step, with a yield of 75.8%.

Ms: 376.1 [M+Na]⁺. ¹H NMR (300 MHz, CDCl₃) δ 3.09 (d, *J* = 6.3 Hz, 2H), 2.70 (s, 3H), 2.02 - 1.91 (m, 2H), 1.71-1.67 (m, 2H), 1.52 - 1.39 (m, 13H), 1.19 - 1.01 (m, 2H).

### Step 6: Synthesis of compound 8

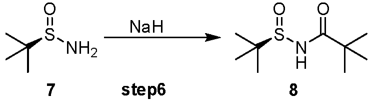

Compound 7 (35 g, 0.289 mol) and anhydrous tetrahydrofuran (1100 ml) were added into a 5L three-necked flask at room temperature, and stirring was started. The temperature was lowered to 0-5°C, sodium hydride (27.5 g, 0.687 mol) was added in batches, and exotherm was intense; after completion of the addition, a THF solution (1000 ml) of pivalic anhydride (51.2 g, 0.275 mol) was added dropwise while the temperature was controlled to be less than 20°C, and the reaction was carried out at room temperature for 2 h. Then 100 mL of methanol was added to the system, and 600 mL of NH₄Cl aqueous solution and 600 mL of saturated brine were added dropwise while the temperature was controlled at 0-20°C to quench the reaction. Then the mixture was extracted with ethyl acetate (500 mL×3), and the organic phase was washed with 1L saturated NaCl, drying and concentrating to obtain an oily substance. Column chromatography PE/EA=5/1 to 3/1 was performed to obtain a solid, which was slurried with 50 mL of petroleum ether to obtain 45.8 g of a white solid with a yield of 81.3%.

Ms: 204.1 [M-H]⁻. ¹H NMR (300 MHz, CDCl₃) δ 6.82 (s, 1H), 1.27 (s, 9H), 1.26 (s, 9H).

### Step 7: Synthesis of compound 9

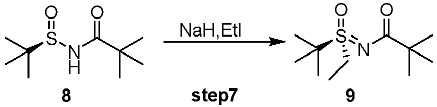

Compound 8 (45 g, 0.22 mol), 1,4-dioxane (200 ml), and 15-crown ether-5 (58 g) were added into a 500 mL three-necked flask in sequence at room temperature, and stirring was started. The temperature was lowered to 10-20°C, sodium hydride (10.5 g, 0.26 mol) was added in batches, and then iodoethane (68.4 g, 1.44 mol) was added dropwise at room temperature after the addition; after the addition, the mixture was stirred at 40°C overnight. Then, aqueous ammonium chloride solution (100 mL) was added to the system for quenching. The aqueous phase was extracted with ethyl acetate (100 mL×3), then washing with 200 mL of saturated NaCl, drying and concentrating to obtain an oily substance. Column chromatography was performed with PE/EA=10/1, 7/1, 5/1, and 3/1 to obtain 45.26 g of a white solid with a yield of 88.3%.

Ms: 234.0 [M+H]⁺.

### Step 8: Synthesis of compound 10

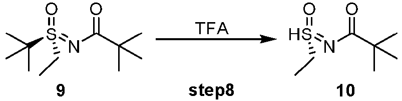

Compound 9 (45 g, 0.19 mol) and dichloromethane (200 ml) were added into a 1L three-necked flask at room temperature, and stirring was started. Trifluoroacetic acid (33 g, 0.29 mol) was added at room temperature; after completion of the addition, the mixture was stirred at room temperature for 3 h. Then saturated aqueous sodium chloride solution (100 mL) was added to the system for quenching. The mixture was extracted with dichloromethane (50 mL×3), then washing with 100 ml of saturated sodium bicarbonate, drying and concentrating to obtain a crude solid. Column chromatography was performed with PE/EA=5/1, 3/1 and 1/1 respectively, to obtain 27 g of a white solid with a yield of 80.2%.

Ms: 178.0 [M+H]⁺.

### Step 9: Synthesis of compound 11

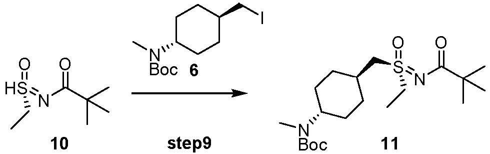

Compound 10 (26 g, 0.146 mol), 1,4-dioxane (146 ml), and 15-crown ether-5 (48.5 g) were added into a 500 mL three-necked flask in sequence at room temperature, and stirring was started. The temperature was lowered to 10-20°C, and sodium hydride (8.8 g, 0.22 mol) was added in batches; after the addition, a THF solution (50 ml) of compound 6 (103.8 g, 0.293 mol) was added dropwise at room temperature. After the addition, the system was stirred at 50°C for 5 days, and then 100 mL of aqueous ammonium chloride solution was added to the system for quenching. Then the reaction mixture was extracted with EA (100 mL×3), and the organic phase was washed with saturated NaCl (200 ml), drying, and concentrating to obtain an oily substance. Column chromatography was performed by eluting with PE/EA=10/1, 7/1, 5/1, and 3/1 in sequence to obtain 31.4 g of a white solid with a yield of 53.5%.

Ms: 402.9 [M+H]⁺. ¹H NMR (300 MHz, CDCl₃) δ 3.61-3.50 (m, 2H), 3.44 - 3.39 (m, 1H), 3.12-3.05 (m, 1H), 2.79 (s, 3H), 2.20-2.10 (m, 3H), 1.82-1.78 (m, 2H), 1.62-1.53 (m, 11H), 1.43 (t, *J* = 7.4 Hz, 3H), 1.36 - 1.24 (m, 11H). Ms: M-56=402.9.

### Step 10: Synthesis of compound 12

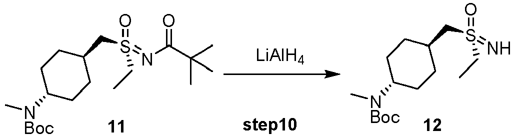

Compound 11 (31 g, 0.077 mol) and anhydrous tetrahydrofuran (500 mL) were added into a 1L three-necked flask, the temperature was controlled at 10°C in an ice bath, and stirring was started under the protection of N₂. Lithium aluminum tetrahydride (8.8 g, 0.24 mol) was added in batches; after the addition, the reaction was carried out for 1 h, and a saturated sodium sulfate solution (24 ml) was added dropwise while controlling the temperature at 0-10°C to quench the reaction. Then 200 ml of ethyl acetate was added to dilute the mixture, filtering with suction under reduced pressure. The filter cake was rinsed with 50 mL of ethyl acetate; after filtering with suction, the filtrate was collected, and 100 ml of water was added for liquid separation. The aqueous phase was extracted with ethyl acetate (50 ml × 2), and the organic phases were combined to wash with saturated sodium chloride solution (100 ml × 1), then drying and concentrating to obtain a colorless oil. Column chromatography was performed by eluting with ethyl acetate to obtain 18.5 g of an oily substance with a yield of 80%.

Ms: 319.3 [M+H]⁺. ¹H NMR (300 MHz, CDCl₃) δ 3.12-3.09 (m, 2H), 2.98-2.95 (m, 2H), 2.76 (s, 3H), 2.62 (s, 1H), 2.22-2.18(m, 1H), 2.12-2.08 (m, 2H), 1.80-1.76 (m, 2H), 1.63-1.43 (m, 14H), 1.33-1.28 (m, 2H).

### Step 11: Synthesis of compound 13

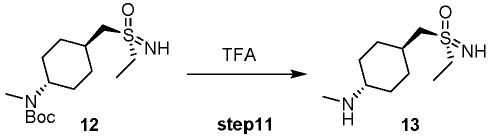

Compound 12 (18.5 g, 0.058 mol) and dichloromethane (45 ml) were added into a 250 mL three-necked flask at room temperature, and stirring was started. Trifluoroacetic acid (20 ml) was added at room temperature; after the addition was completed, the mixture was stirred at room temperature overnight, then concentrating until no fraction was evaporated to obtain 13.3 g of a crude product, which was directly used in the next step.

Ms: 219.2 [M+H]⁺.

### Step 12: Synthesis of compound 15

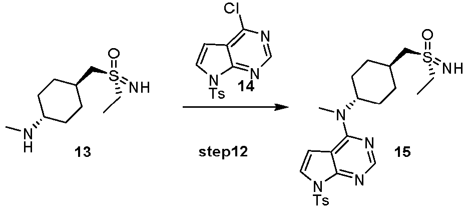

Compound 13 (13.3 g, 0.058 mol), anhydrous DMF (100 ml), potassium carbonate (33.4 g, 0.24 mol) and compound 14 (17.8 g, 0.058 mol) were added into a 250 mL three-necked flask in sequence, and stirring was started. The temperature was raised to 115°C, and the reaction was carried out overnight. The temperature was lowered to room temperature, and then water (150 ml) was added to quench the reaction. The mixture was extracted with ethyl acetate (100 mL × 6), then washing with saturated NaCl (500 ml × 3). The organic phase was dried and concentrated to obtain an oily substance. Column chromatography was performed with PE/EA = 1/1, DCM/MeOH = 25/1 to obtain a semi-solid substance, then adding 10 ml of ethyl acetate and 50 ml of n-hexane to slurry to obtain 22.4 g of an off-white solid with a yield of 76.5%.

Ms: 489.7 [M+H]⁺.

### Step 13: Synthesis of Compound T1

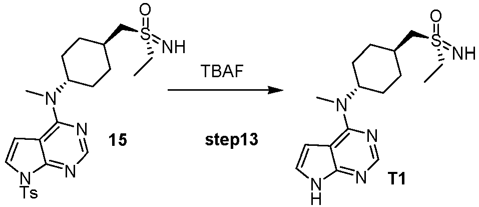

Compound 15 (22.4 g, 0.045 mol), anhydrous THF (250 ml) and TBAF solution (93 ml, 1 M/L) were added into a 500 mL three-necked flask, and stirring was started. The mixture was refluxed to react overnight, and the reaction solution was concentrated while decreasing the temperature until no fraction was evaporated. Water (150 ml) and ethyl acetate (200 ml) were then added to the system in sequence to separate the organic phase. The aqueous phase was extracted with ethyl acetate (100 ml × 3), washing with saturated NaCl (500 ml × 1), drying over anhydrous sodium sulfate, and concentrating to obtain a solid crude product. The product was separated by column chromatography, eluting with DCM/MeOH = 25/1, then slurrying with ethyl acetate (20 ml) to obtain 11 g (purity: 99.1%) of a white solid with a yield of 73.3%.

Ms: 336.2 [M+H]⁺. ¹H NMR (400 MHz, DMSO) δ 11.60 (s, 1H), 8.08 (s, 1H), 7.11 (s, 1H), 6.53 (s, 1H), 4.67 (s, 1H), 3.57 (s, 1H), 3.16 (s, 3H), 2.97 (q, *J* = 7.6 Hz, 2H), 2.93(d, *J* = 6.0 Hz, 2H), 2.12-1.99 (m, 3H), 1.75-1.68 (m, 4H), 1.33-1.24 (m, 2H), 1.22 (t, *J* = 7.6 Hz, 3H).

It can be seen that, compared with CN111499641A and other traditional preparation methods, the steps and operations of Example 1 are simple, relatively safe, the target product has a higher purity, and the yield is 2-4 times higher than the yield in the preparation method disclosed in CN111499641A.

### Example 2

The synthetic scheme is as follows:

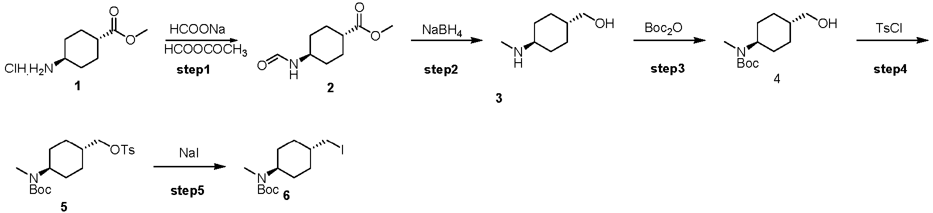

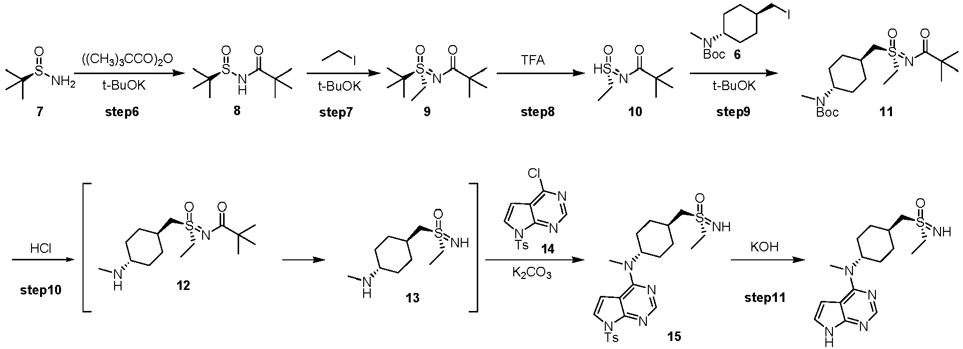

### Step 1:

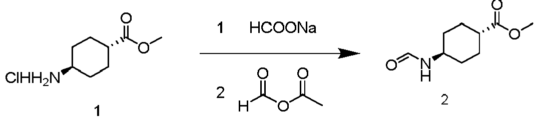

Formic acid (12 Kg, 260.7 mol) was added into a reactor, and acetic anhydride (21.2 kg, 207.7 mol) was added dropwise at room temperature while controlling the temperature not to exceed 40°C. After completion of the addition, the temperature was raised to 50-55°C, keeping warm for 2h. Then heating was stopped to cool to room temperature. Tetrahydrofuran (40 L), sodium formate (3.9 Kg, 57.34 mol) and methyl trans-4-(amino)cyclohexanecarboxylate hydrochloride (10 Kg, 51.6 mol) were added in sequence, and the temperature was raised to 50-55°C, keeping warm for 16 h. Samples were taken to perform in-process control, then cooling to room temperature after completion of the reaction, filtering with suction through celite pad, rinsing the solid with tetrahydrofuran (about 10 L), and combining the mother liquors to concentrate under reduced pressure at 30-60°C to obtain about 21 kg of a light yellow oily liquid crude product, which was directly used in the next step reaction.

Ms: 186 [M+H]⁺.

### Step 2:

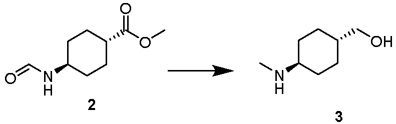

The crude product of reaction material 2 from the previous step (the product obtained in step 1) (10.38 Kg, 26.56 mol) and tetrahydrofuran (50 L) was added into a reaction flask, cooling to 5-10°C in an ice bath. Sodium borohydride (6.0 Kg, 159.36 mol) was added in batches while controlling the temperature not to exceed 20°C; after completion of the addition, the temperature was slowly raised to 25°C, keeping warm for 12 h. After cooling down to -5°C, ether solution of boron trifluoride (21.7Kg, 159.36mol) was added dropwise within about 7h, and the temperature was controlled at 0-20°C while adding. After completion of the addition, the button of the high and low temperature integrated machine was adjusted to raise the temperature to 40°C, then keeping warm overnight. Samples were taken to perform in-process control until the raw materials were reacted completely. The reaction liquid was discharged from the reactor, and 20L of water and 5.5L of hydrochloric acid were added into the reactor to stir the mixture. The temperature was lowered to 0°C, and the reaction liquid was added into the reactor in small amounts in batches for quenching, while controlling the temperature not to exceed 20°C. After completion of the addition, the temperature was raised to 35°C, and the mixture was stirred for 12 hours. After stopping the stirring, the reaction liquid was discharged to concentrate under reduced pressure until no tetrahydrofuran dripped out. Then the concentrated liquid was poured back into the reactor, and 15L of water was added to stir and cool to 0°C. 6KG of sodium hydroxide was dissolved in 20L of water, and the mixture was poured into the reactor in batches while controlling the temperature not to exceed 30°C, adjusting the pH to 12. The reaction liquid was then discharged to filter with suction, and the solid was washed with water (about 10L), combining the aqueous phases which were directly used for the next reaction.

Ms: 144 [M+H]⁺.

### Step 3:

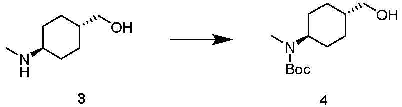

An aqueous solution of the reaction material from the previous step was added into a reactor, then adding 15L of dichloromethane, and adding BOC anhydride (5.6Kg, 25.56mol) dropwise at room temperature while controlling the temperature not to exceed 30°C; after completion of the addition, the mixture was stirred at room temperature for 30min, then raising the temperature to 30-35°C, and keeping the temperature for 5h. The samples were taken to perform in-process control until the raw materials were reacted completely. The reaction mixture were separated into layers, and the aqueous phase was extracted with dichloromethane (10L); the organic phases were combined, then adding water (5L×2) for extraction and washing, and stirring for 10min. The mixture was kept still to separate into layers, and the organic phases were collected to dry over anhydrous sodium sulfate, then concentrating to obtain 6.1kg of crude product, which was directly used in the next step, with the total yield of 94% in the three steps.

Ms: 244 [M+H]⁺.

### Step 4:

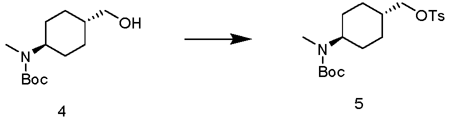

Material 4 (the crude product obtained in step 3) (6.1 Kg, 25.1 mol) and dichloromethane (30 L) were added into a reactor, then adding triethylamine (5 Kg, 50.2 mol) at room temperature and stirring at room temperature for 30 min, adding a dichloromethane solution (10 L) of TsCl dropwise at room temperature while controlling the temperature not to exceed 30°C; after completion of the addition, the reaction mixture was stirred at room temperature overnight until the reaction raw materials disappeared, then stopping the reaction. Water was added to wash twice (10 L × 2), collecting the organic phase to dry over anhydrous sodium sulfate, and then concentrating until there was no dichloromethane. The concentrated crude product was added to n-hexane (40 L) to stir for 30 min, then filtering with suction, rinsing the solid with n-hexane (10 L) and drying to obtain 7.6 kg of white product (yield: 76.0%, purity>95%).

Ms: 420 [M+Na]⁺. ¹H NMR (400 MHz, DMSO) δ 7.78 (d, *J =* 8.4 Hz, 2H), 7.48 (d, *J =* 8.0 Hz, 2H), 3.84 (d, *J* = 6.3 Hz, 2H), 3.10-3.07 (m, 1H), 2.62 (s, 3H), 2.43 (s, 3H), 1.69-1.65 (m, 2H), 1.54-1.50 (m, 3H), 1.38 (s, 9H), 1.20-1.68 (m, 2H), 1.04-0.91 (m, 2H).

### Step 5:

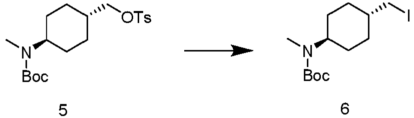

Acetone (70L), material 5 (product obtained in step 4) (7Kg, 17.6mol) and sodium iodide (5.3Kg, 35.2mol) were added into a reaction flask, then raising the temperature to reflux, and keeping warm for 16h. Samples were taken to perform in-process control until the raw materials were reacted completely, cooling down, and concentrating to recover acetone. Water (25 L) and ethyl acetate (15 L) were added to stir for 30 min, separating the aqueous layer to extract with ethyl acetate (10 L × 2), and combining the organic phases to dry over anhydrous sodium sulfate, then concentrating to evaporate the solvent to obtain 5.8 Kg of product (yield: 93.5%, purity: 98%).

Ms: 339 [M+H-15]⁺. ¹H NMR (400 MHz, CDCl₃) δ 3.09 (d, *J* = 6.3 Hz, 2H), 2.71 (s, 3H), 1.98 (d, *J =* 12.8 Hz, 2H), 1.70 (d, *J* = 11.6 Hz, 2H), 1.51-1.36 (m, 13H), 1.16-1.08 (m, 2H).

### Step 6:

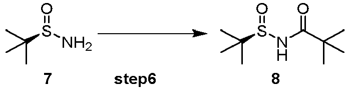

Tert-butanol (70L) and potassium tert-butoxide (12.75Kg, 113.63mol) were added into a reaction flask to stir at room temperature for 1h, then raising the temperature to 35-40°C and adding material 7 (product obtained in step 5) (5.5Kg, 45.45mol) in batches, and controlling the temperature not to exceed 40°C to continue stirring for 1h. Pivalic anhydride (10.15Kg, 54.54mol) was added dropwise while controlling the temperature not to exceed 40°C; after completion of the addition, the mixture was kept warm for 2h, and in-process control was performed by TLC until the raw material disappeared. The mixture was cooled to room temperature, 2M hydrochloric acid (4 L of concentrated hydrochloric acid was added to 20 L of water) was added dropwise to adjust the pH to 7-8, and the solvent tert-butanol was removed by concentration under reduced pressure. Water (50 L) was added to stir at room temperature for 1 h, then filtering with suction. The filter cake was added into 20 L of dichloromethane to dissolve, then adding anhydrous sodium sulfate to dry it, and concentrating under reduced pressure to obtain 5.1 kg of solid product. The filtrate was extracted twice with dichloromethane (10 L x 2), and the organic phases were collected to dry over anhydrous sodium sulfate, then concentrating under reduced pressure to obtain 3 kg of the product. A total of 8.1 kg of solid product was obtained after combination (yield: 87.0%, purity>95%).

Ms: 206 [M+H]⁺. ¹H NMR (400 MHz, CDCl₃) δ 6.90 (s, 1H), 1.27 (s, 9H), 1.25 (s, 9H).

### Step 7:

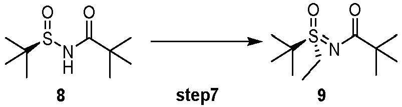

Dioxane (45L), material 8 (product obtained in step 6) (1.6Kg, 7.8mol) and 18-crown 6 (3.1Kg, 11.7mol) were added into a reaction flask in sequence, stirring at room temperature for 1h, adding potassium tert-butoxide (1.3Kg, 11.7mol) in batches while controlling the temperature not to exceed 40°C, keeping warm at 35-40°C for 2h, and adding iodine ethane (2.5Kg, 15.6mol) dropwise; after completion of the addition, the reaction mixture was kept warm at 40-45°C for 16h, and samples were taken to perform in-process control. When the reaction of the raw materials was completed, stopping the reaction, concentrating under reduced pressure to remove dioxane, then adding ethyl acetate (25L) and 20% ammonium chloride solution (15L) to extract and separate into layers; the aqueous phase was extracted twice with ethyl acetate (5L×2), combining the organic phases, and washing with 15L of saturated brine. The organic phases were collected to dry over anhydrous sodium sulfate. The ethyl acetate was recovered by concentration until almost no solvent was collected, and a brown-yellow solid was obtained, n-hexane (5 L) was added to the solid to stir for 1 h, then filtering; and the solid was washed with n-hexane (1 L). The solid was dried to obtain 1.1 kg of the product. The mother liquor was concentrated until there was no solvent, and n-hexane (1.5 L) was added to stir to precipitate a solid, then filtering to obtain 0.2 kg of the product. A total of 1.3 kg of the product was obtained (yield: 71.5%, purity>95%).

Ms: 234 [M+H]⁺. ¹H NMR (400 MHz, CDCl₃) δ 3.69-3.58 (m, 1H), 3.43-3.36 (m, 1H), 1.47 (s, 9H), 1.46 (t, *J=* 7.6 Hz, 3H), 1.20 (s, 9H).

### Step 8:

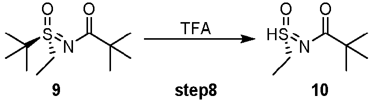

Dichloromethane (21.5L) and material 9 (the product obtained in step 7) (4.3Kg, 18.45mol) were added into a reaction flask, and trifluoroacetic acid (2.31Kg, 20.3mol) was added dropwise at room temperature. After completion of the addition, the mixture was kept warm for 2h, and samples were taken to perform in-process control until the raw material reaction was completed. Then sodium carbonate aqueous solution (2Kg sodium carbonate was dissolved in 10L water) was added to adjust the pH to 8-9, then stirring for 0.5h, and allowing to stand still to separate into layers. The organic phase was collected, 10L saturated sodium bicarbonate solution was added, then stirring for 0.5h, and allowing to stand still to separate into layers. The organic phase was collected, and the aqueous phase was extracted twice with an appropriate amount of dichloromethane (5L×2). The organic phases were combined to dry over anhydrous sodium sulfate, and dichloromethane was recovered by rotary evaporation. The solid was rotary dried to obtain 2.64 kg of the product (yield: 80.8%, purity>95%).

Ms: 178 [M+H]⁺. ¹H NMR (400 MHz, CDCl₃) δ 3.14-3.04 (m, 2H), 1.29 (t, *J =* 7.6 Hz, 3H), 1.22 (s, 9H).

### Step 9:

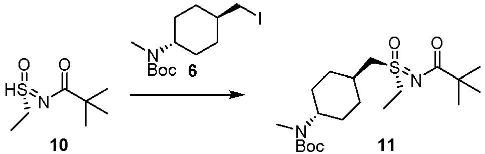

Dioxane (60L), material 10 (the product obtained in step 8) (2Kg, 11.3mol) and 18-crown 6 (3.58Kg, 13.56mol) were added into a reaction flask, then stirring at room temperature for 30min. Potassium tert-butoxide (1.5Kg, 13.56mol) was added in batches while controlling the temperature not to exceed 40°C, then keeping at 35-40°C for 2h. Material 6 (the product obtained in step 5) (4.4kg, 12.4mol) was added, then raising the temperature to 60°C and keeping warm for 7d, and then stopping heating and concentrating the reaction solution under reduced pressure until there was no dioxane. Ethyl acetate (20 L) and 20% ammonium chloride solution (25 L) were added to extract and separate into layers. The aqueous phase was extracted once more with ethyl acetate (10 L). The organic phases were combined, washing with saturated brine (15 L), and drying over anhydrous sodium sulfate. The ethyl acetate was recovered by concentration until almost no solvent was collected, and a brown-yellow oil was obtained, then pouring it into a reactor, adding an appropriate amount of n-hexane (40L) to stir and cool to 0-5°C, stirring for 4h, filtering and washing the solid with n-hexane (2L), and then drying the solid to obtain a solid product. The mother liquor was concentrated under reduced pressure to obtain an oily substance, which was separated by n-hexane/ethyl acetate column chromatography to obtain some solid products. All the products were combined to obtain 2.05 Kg (yield: 45%, purity: 98%).

Ms 403 [M+H]⁺; ¹H NMR (400 MHz, CDCl₃) δ 4.05 - 3.74 (m, 1H), 3.56 - 3.39 (m, 2H), 3.37 - 3.26 (m, 1H), 2.99 (dd, *J* = 13.7, 5.9 Hz, 1H), 2.70 (s, 3H), 2.14 - 1.91 (m, 3H), 1.71 (dt, *J =* 9.8, 3.0 Hz, 2H), 1.54 - 1.45 (m, 2H), 1.44 (s, 9H), 1.34 (t, *J* = 7.4 Hz, 3H), 1.29 - 1.22 (m, 2H), 1.19 (s, 9H).

### Step 10:

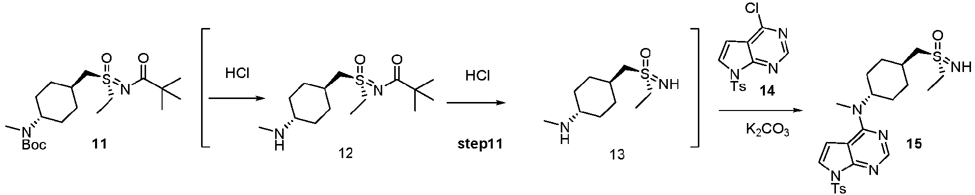

Hydrochloric acid was added into a reaction flask, then cooling it in an ice water bath, adding material 11 (the product obtained in step 9) in batches and keeping warm for 30 minutes, and then raising the temperature to 55-60°C and keeping warm for 5 hours. Samples were taken to perform in-process control until the reaction of material 11 was basically completed, then cooling to room temperature, adding appropriate amount of water, and adjusting the aqueous phase to pH = 7-8 with appropriate amount of potassium carbonate. Appropriate amounts of potassium carbonate, tetrahydrofuran and compound 14 were added, then raising the temperature to 60-65°C and keeping warm to react for 16 hours. Samples were taken to perform in-process control until the reaction of compound 13 was basically completed. Then the temperature was lowered to separate into the layers, the aqueous layer was extracted with an appropriate amount of tetrahydrofuran, and the organic phases were combined to concentrate by rotary evaporation. After most of the solvent was removed, an appropriate amount of ethyl acetate was added to dissolve the solid, the temperature was lowered to perform crystallization, then filtering with suction, and drying the solid to obtain 7.0 g of the product with a yield of 72.0%.

Ms: 490 [M+H]⁺. ¹H NMR (400 MHz, CDCl₃) δ 8.37 (s, 1H), 8.05 (d, *J* = 8.4 Hz, 2H), 7.44 (d, *J=* 4.0 Hz, 1H), 7.28 (d, *J* = 8.4 Hz, 2H), 6.60 (d, *J* = 4.0 Hz, 1H), 3.14 (s, 3H), 3.10-3.04 (m, 2H), 2.99-2.89 (m, 2H), 2.38 (s, 3H), 2.25-2.09 (m, 4H), 1.82-1.79 (m, 2H), 1.69-1.63 (m, 2H), 1.44-1.36 (m, 5H).

### Step 11:

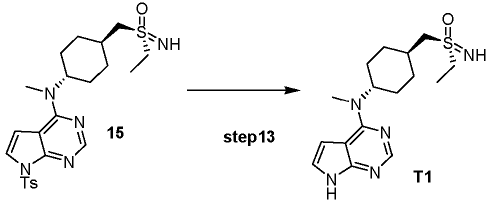

Tert-butanol, potassium hydroxide, and material 15 (the product obtained in step 10) were added into a reaction flask, then stirring, heating to 60°C and keeping warm for 3 hours. Samples were taken to perform in-process control, after the reaction of the raw materials was completed, the reaction mixture was filtered while hot, the mother liquor was spin-dried, adding appropriate amounts of dichloromethane and water to extract and separate into layers. The aqueous layer was extracted with appropriate amount of dichloromethane, then combining the organic phases to wash with water, drying over anhydrous sodium sulfate, spin-drying, slurrying with appropriate amount of ethyl acetate, and then filtering with suction, and drying the solid to obtain 2.1 g of product. The aqueous phase was adjusted to a neutral pH with hydrochloric acid, then extracting again with dichloromethane, drying, and spin-drying to obtain 1.0 g of the product. A total of 3.1 g of product was obtained (purity: 98.4%), with a total yield of 88.7%.

Ms: 336 [M+H]⁺. ¹H NMR (400 MHz, DMSO) δ 11.60 (s, 1H), 8.09 (s, 1H), 7.11 (s, 1H), 6.53 (s, 1H), 3.32-3.31 (m, 1H), 3.16 (s, 3H), 3.01 (q, *J* = 7.4 Hz, 2H), 2.94 (d, *J* = 5.9 Hz, 2H), 2.17-2.08 (m, 1H), 2.04-1.95 (m, 2H), 1.73-1.68 (m, 4H), 1.30-1.27 (m, 2H), 1.23 (t, *J =* 7.4 Hz, 3H).

Compared with CN111499641A and other traditional preparation methods, the reagents used in Example 2 are stable, readily available, non-toxic, the reaction conditions are milder, the steps are simpler, the operability of the reaction is further improved, the cost is lower, the purity of the target product is higher, and the yield is higher (equivalent to 3-5 times the yield of the preparation method disclosed in CN111499641A), the method of this example is suitable for industrial scale-up production.

### Example 3

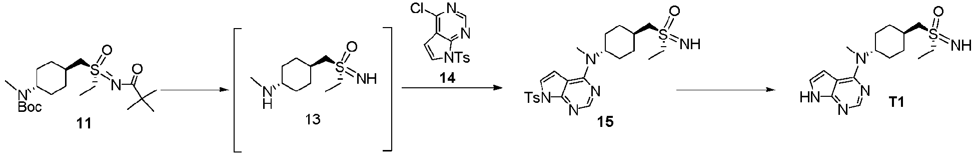

### Step 1:

Compound 11 (as prepared in Examples 1 and 2) and 3N HCl solution were stirred at 20-30°C for 1.5 hours and at 55-60°C for 5 hours. Then potassium carbonate was used to adjust the pH of the reaction solution to 7-8, stirring at 25-30°C for 20 minutes, K₂CO₃, compound 14 and tetrahydrofuran were added to the solution, then stirring at 60-65°C for 16 hours. Then the reaction product was concentrated at 35-40°C, adjusting to pH 3-4 with 3N HCl, and stirring for 20 minutes. Ethyl acetate was used to extract impurities in the solution. The aqueous solution was adjusted to pH 9-10 with K₂CO₃ and extracted twice with dichloromethane. The organic layer was concentrated to remove DCM, and the residue was crystallized with EA to obtain compound 15 as an off-white powder, with a yield of 75%.

¹H NMR (600 MHz, CDCl₃) δ 8.37 (s, 1H), 8.04 (d, *J* = 8.4 Hz, 2H), 7.43 (d, *J* = 4.2 Hz, 1H), 7.28 (s, 2H), 6.61 (s, 1H), 4.69 (s, 1H), 3.14 (s, 3H), 3.10 - 3.03 (m, 2H), 2.95 (qd, *J* = 13.7, 5.9 Hz, 2H), 2.40 - 2.34 (m, 4H), 2.25 - 2.19 (m, 1H), 2.15 - 2.08 (m, 2H), 1.83 - 1.76 (m, 2H), 1.71 - 1.62 (m, 2H), 1.44 - 1.35 (m, 5H). ¹³C NMR (151 MHz, CDCl₃) δ 157.21, 152.91, 151.85, 145.32, 135.15, 129.63, 128.15, 120.97, 105.76, 104.84, 59.45, 54.29, 51.08, 32.43, 32.18, 31.98, 31.39, 29.11, 29.02, 21.64, 7.18.

### Step 2:

A solution of compound 15, potassium hydroxide and water was stirred at 60-65°C for 16 hours. Then the reaction product was diluted with DCM, adjusting pH to 2-3 with 3N HCl and stirring at 25-30 °C for 30 min. The pH of the solution was adjusted to 9-10 with K₂CO₃, then stirring at 25-30°C for 30 minutes. After the mixture was separated into layers, the organic layer was collected, and the aqueous layer was extracted twice with dichloromethane. The combined organic layers were concentrated to remove DCM, and the residue was crystallized with EtOH to obtain compound T1 (purity: 99.6%) as an off-white powder, with a yield of 72%.

MS: 336.18 [M+H]⁺; ¹H NMR (600 MHz, MeOD) δ 8.08 (s, 1H), 7.08 (d, *J =* 3.6 Hz, 1H), 6.61 (d, *J =* 3.7 Hz, 1H), 4.70 (s, 1H), 3.24 (s, 3H), 3.15 (qd, *J =* 7.2, 2.5 Hz, 2H), 3.07 (qd, *J =* 14.1, 5.9 Hz, 2H), 2.22 (d, *J =* 12.9 Hz, 1H), 2.14 - 2.06 (m, 2H), 1.86 - 1.77 (m, 4H), 1.45 - 1.39 (m, 2H), 1.38 (t, *J* = 7.4 Hz, 3H). ¹³C NMR (151 MHz, CDCl₃) δ 157.44, 151.78, 150.91, 119.91, 102.88, 102.10, 59.63, 54.14, 51.05, 32.60, 32.35, 32.11, 31.16, 29.34, 29.25, 7.21.

On the basis of Example 2, the reaction conditions of Example 3 are milder, the operation is simpler, the cost is lower, and the quality and purity of the target product are very high, the method of this example is more conducive to industrial scale-up production.

The above description is only preferred examples of the present application, and is not intended to limit the present application. Any modifications, equivalent substitutions, etc. made within the spirit and principle of the present application should be included in the protection scope of the present application.

The aforementioned examples and methods described in the present application may be varied based on the ability, experience and preference of those skilled in the art.

In the present application, the steps of a method are merely listed in a certain order, and they do not constitute any limitation on the order of the steps of the method.

## Claims

1. A preparation method of a stereoisomer comprises the following steps:
(1) mixing compound RM01 with solvent 1, catalyst 1, a base and compound RM02 to react to obtain compound RM03, 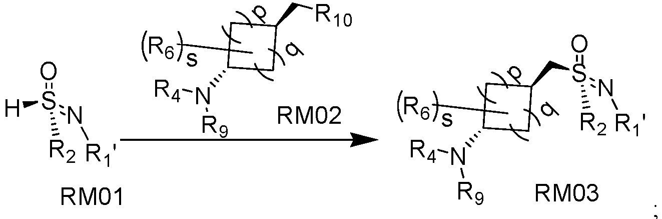
(2) deprotecting the amino group of compound RM03 to obtain compound RM04, 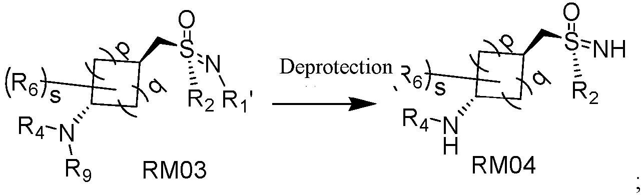 and
(3) mixing compound RM04 with solvent 3, a base, and compound RM05 to react to obtain compound RM06, 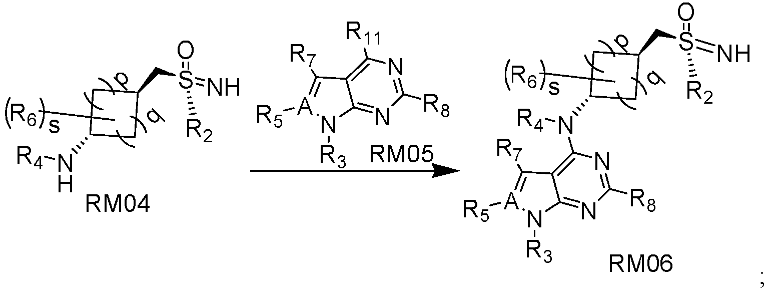
wherein A is selected from C or N; when A is N, R₅ is absent, and when A is C, R₅ is selected from the group consisting of: H, halogen, hydroxyl, cyano, substituted or unsubstituted alkyl, substituted or unsubstituted haloalkyl, substituted or unsubstituted alkoxy, substituted or unsubstituted hydroxyalkyl, substituted or unsubstituted cycloalkyl, substituted or unsubstituted heterocycloalkyl, substituted or unsubstituted amino, substituted or unsubstituted sulfonic acid group, and substituted or unsubstituted sulfonyl;
R₁' is an amino protecting group;
R₂ is selected from the group consisting of: halogen, -CN, -OCH₂F, -OCHF₂, -OCF₃, C₁₋₁₀ alkyl, C₃₋₁₀ cycloalkyl, -OC₀₋₁₀ alkyl, -N(C₀₋₁₀ alkyl)(C₀₋₁₀ alkyl), -O heterocycloalkyl, -N heterocycloalkyl, -S heterocycloalkyl, C₆₋₁₈ aryl, -N heterocyclic aryl, -S heterocyclic aryl or -O heterocyclic aryl, cycloalkylalkyl, aralkyl and heterocyclylalkyl, wherein the H on the carbon or nitrogen atom may be substituted by a group selected from: deuteration, hydroxyl, halogen, -CN, -OCH₂F, -OCHF₂, -OCF₃, C₁₋₆ straight chain alkyl, -N(C₀₋₁₀ alkyl)(C₀₋₁₀ alkyl), -OC₀₋₁₀ alkyl, C₃₋₁₀ cycloalkyl, -O heterocycloalkyl, -N heterocycloalkyl, -S heterocycloalkyl, C₆₋₁₈ aryl, -N heterocyclic aryl, -O heterocyclic aryl or -S heterocyclic aryl; and wherein the H on the C₆₋₁₈ aryl or heterocyclic aryl may be substituted by any one or more groups selected from: halogen, C₁₋₄ straight chain alkyl, -N(C₀₋₁₀ alkyl)SO₂(C₀₋₁₀ alkyl), -CON(C₀₋₁₀ alkyl)(C₀₋₁₀ alkyl), -N(C₀₋₁₀ alkyl)CO(C₀₋₁₀ alkyl), -N(C₀₋₁₀ alkyl)COO(C₀₋₁₀ alkyl), -OCON(C₀₋₁₀ alkyl)(C₀₋₁₀ alkyl), -CN, -OCH₂F, -OCHF₂, -OCF₃, -N(C₀₋₁₀ alkyl)(C₀₋₁₀ alkyl), -OC₀₋₁₀ alkyl, -N heterocyclic aryl, -O heterocyclic aryl or -S heterocyclic aryl; or the adjacent carbon atoms on the C₆₋₁₈ aryl or heterocyclic aryl form a C₃₋₈ cycloalkyl, -O heterocycloalkyl, -N heterocycloalkyl, -S heterocycloalkyl, -N heterocyclic aryl, -O heterocyclic aryl, or -S heterocyclic aryl;
R₃ is an amino protecting group;
R₄ is selected from the group consisting of: H, C₁₋₁₀ straight chain/branched alkyl, C₁₋₁₀ straight chain/branched alkenyl, C₁₋₁₀ straight chain/branched alkynyl, C₆₋₁₈ aryl, C₆₋₁₈ heterocyclic aryl, C₃₋₁₀ cycloalkyl, -OC₀₋₁₀ alkyl and -O heterocycloalkyl, wherein the H on the carbon atom may be substituted by a group selected from: deuteration, hydroxyl, halogen, -CN, -OCH₂F, -OCHF₂, -OCF₃, C₁₋₁₀ straight chain/branched alkyl, -N(C₀₋₁₀ alkyl)(C₀₋₁₀ alkyl), -OC₀₋₁₀ alkyl, C₃₋₁₀ cycloalkyl, -O heterocycloalkyl, -N heterocycloalkyl, -S heterocycloalkyl, C₆₋₁₈ aryl, -N heterocyclic aryl, -O heterocyclic aryl or -S heterocyclic aryl; and wherein the alkyl moiety of the group is substituted by any one or more groups selected from: -SO₂, -SO₂N(C₀₋₁₀ alkyl)(C₀₋₁₀ alkyl), -N(C₀₋₁₀ alkyl)SO₂(C₀₋₁₀ alkyl), -CON(C₀₋₁₀ alkyl)(C₀₋₁₀ alkyl), -N(C₀₋₁₀ alkyl)CO(C₀₋₁₀ alkyl), -N(C₀₋₁₀ alkyl)COO(C₀₋₁₀ alkyl), -OCON(C₀₋₁₀ alkyl)(C₀₋₁₀ alkyl), halogen, -CN, -OCH₂F, -OCHF₂, -OCF₃, -N(C₀₋₁₀ alkyl)(C₀₋₁₀ alkyl), -OC₀₋₁₀ alkyl, C₆₋₁₈ aryl, -N heterocyclic aryl, -O heterocyclic aryl or -S heterocyclic aryl;
R₆ is a substituent group for H on one or more carbon atoms on a cycloalkyl group, each R₆ is independently selected from the group consisting of: C₁₋₆ alkyl and C₃₋₆ cycloalkyl, s is an integer from 0-8 (e.g., 0, 1, 2, 3, 4, or 5);
p is any integer from 0-4, q is any integer from 0-4, and p and q are not 0 at the same time;
R₇ and R₈ are independently selected from the group consisting of: H, halogen, hydroxyl, cyano, substituted or unsubstituted alkyl, substituted or unsubstituted alkenyl, substituted or unsubstituted alkynyl, substituted or unsubstituted haloalkyl, substituted or unsubstituted alkoxy, substituted or unsubstituted hydroxyalkyl, substituted or unsubstituted cycloalkyl, substituted or unsubstituted non-heterocyclic aryl, substituted or unsubstituted heterocyclic aryl, substituted or unsubstituted heterocycloalkyl, substituted or unsubstituted amino, substituted or unsubstituted sulfonic acid group, and substituted or unsubstituted sulfonyl;
R₉ is an amino protecting group; and
R₁₀ and R₁₁ are independently selected from halogen;
optionally, the preparation method further comprises the steps of:
(4) deprotecting the amino group of compound RM06 to obtain compound I, 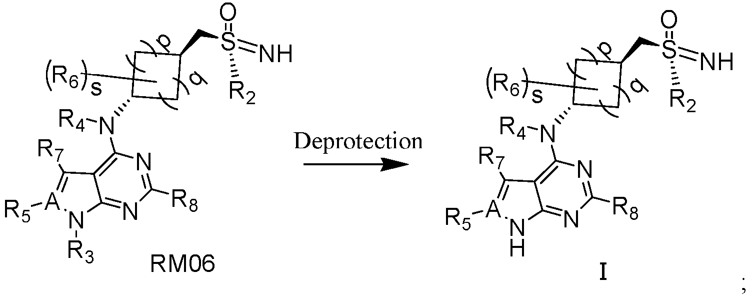 or
(4') reacting compound RM06 with a modifying agent to obtain compound RM07, 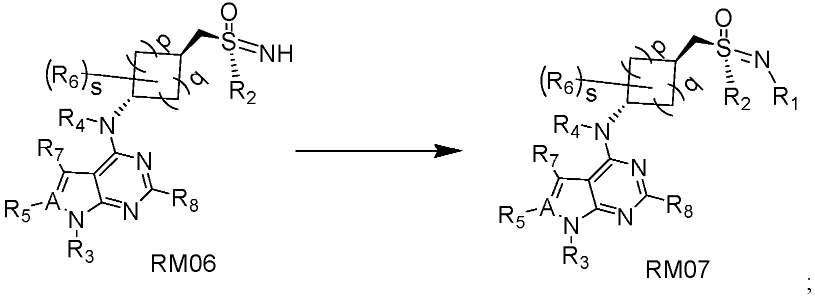
(5') deprotecting the amino group of compound RM07 to obtain compound II, 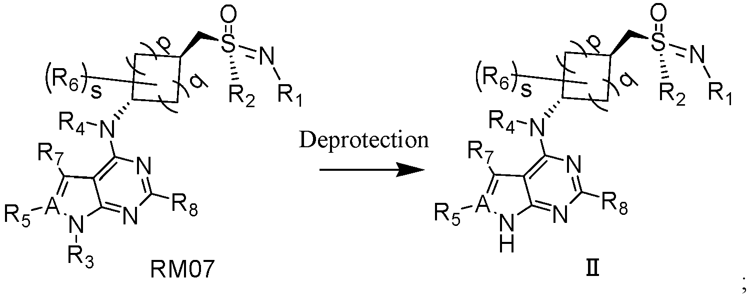 wherein R₁ is selected from the group consisting of: halogen, -CN, -OCH₂F, -OCHF₂, -OCF₃, C₁₋₁₀ alkyl, C₃₋₁₀ cycloalkyl, -OC₀₋₁₀ alkyl, -N(C₀₋₁₀ alkyl)(C₀₋₁₀ alkyl), -O heterocycloalkyl, -N heterocycloalkyl, -S heterocycloalkyl, C₆₋₁₈ aryl, -N heterocyclic aryl, -S heterocyclic aryl or -O heterocyclic aryl, cycloalkylalkyl, aralkyl and heterocyclylalkyl, wherein the H on the carbon or nitrogen atom may be substituted by a group selected from: deuteration, hydroxyl, halogen, -CN, -OCH₂F, -OCHF₂, -OCF₃, C₁₋₆ straight chain alkyl, -N(C₀₋₁₀ alkyl)(C₀₋₁₀ alkyl), -OC₀₋₁₀ alkyl, C₃₋₁₀ cycloalkyl, -O heterocycloalkyl, -N heterocycloalkyl, -S heterocycloalkyl, C₆₋₁₈ aryl, -N heterocyclic aryl, -O heterocyclic aryl or -S heterocyclic aryl; and wherein the H on the C₆₋₁₈ aryl or heterocyclic aryl may be arbitrarily substituted by any one or more groups selected from: halogen, C₁₋₄ straight chain alkyl, -N(C₀₋₁₀ alkyl)SO₂(C₀₋₁₀ alkyl), -CON(C₀₋₁₀ alkyl)(C₀₋₁₀ alkyl), -N(C₀₋₁₀ alkyl)CO(C₀₋₁₀ alkyl), -N(C₀₋₁₀ alkyl)COO(C₀₋₁₀ alkyl), -OCON(C₀₋₁₀ alkyl)(C₀₋₁₀ alkyl), -CN, -OCH₂F, -OCHF₂, -OCF₃, -N(C₀₋₁₀ alkyl)(C₀₋₁₀ alkyl), -OC₀₋₁₀ alkyl, -N heterocyclic aryl, -O heterocyclic aryl or -S heterocyclic aryl; or the adjacent carbon atoms on the C₆₋₁₈ aryl or heterocyclic aryl form a C₃₋₈ cycloalkyl, -O heterocycloalkyl, -N heterocycloalkyl, -S heterocycloalkyl, -N heterocyclic aryl, -O heterocyclic aryl or -S heterocyclic aryl.

2. The preparation method according to claim 1, wherein the solvent 1 in step (1) is any one or more selected from the group consisting of: ethyl acetate, isopropanol, chloroform, methylethyl ketone, dioxane, pyridine, DMF, NMP, tetrahydrofuran and 2-methyltetrahydrofuran, preferably dioxane;
the catalyst 1 in step (1) is a crown ether, preferably 18-crown ether-6 or 15-crown ether-5;
the base in step (1) is a strong base or a weak base, which is selected from the group consisting of: sodium hydride, potassium hydride, potassium amide, sodium amide, sodium methoxide, sodium ethoxide, potassium ethoxide, sodium tert-butoxide, potassium tert-butoxide, butyl lithium, lithium diisopropylamide, benzyl lithium, Grignard reagent, DBU, potassium carbonate, sodium carbonate, potassium bicarbonate, sodium bicarbonate, triethylamine and pyridine, preferably sodium hydride, potassium tert-butoxide or potassium carbonate; and
the reaction temperature in step (1) is 20-70°C, preferably 40-60°C.

3. The preparation method according to claim 1 or 2, wherein R₁' is an acyl amino protecting group, R₉ is an alkoxycarbonyl amino protecting group, and step (2) comprises: deprotecting the amino group of compound RM03 under acidic condition to obtain compound RM04;
preferably, step (2) comprises: adding an acid and compound RM03 to a reaction vessel to react at 50-65°C; and
more preferably, the acid is an inorganic acid, such as hydrochloric acid, sulfuric acid, nitric acid, phosphoric acid, or hydrobromic acid.

4. The preparation method according to any one of claims 1-3, wherein the solvent 3 in step (3) is any one or more selected from the group consisting of: dimethylformamide, methanol, acetic acid, acetonitrile, ethyl acetate, isopropanol, acetone and tetrahydrofuran;
the base in step (3) is a weak base, preferably an inorganic weak base, such as potassium carbonate, sodium carbonate, potassium bicarbonate or sodium bicarbonate; and
the reaction temperature in step (3) is 50-120°C.

5. The preparation method according to any one of claims 1-4, wherein steps (2) and (3) comprise:
(A) mixing compound RM03 with an acid to react at 50-65°C;
(B) cooling, adding water, and adjusting the pH to weak alkalinity; and
(C) adding a base, solvent 3 and compound RM05 to react at 55-70°C.

6. The preparation method according to claim 5, wherein step (A) comprises: mixing compound RM03 with an acid, stirring at 20-30°C, and then stirring at 50-65°C; and/or,
step (B) comprises: adjusting the pH to weak alkalinity, and stirring at 20-30°C.

7. The preparation method according to any one of claims 1-6, wherein the compound RM01 is 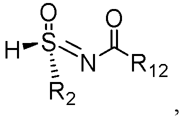 and the preparation method further comprises the following steps:
(a) mixing compound RM01-1 with solvent a, adding a base, and then adding compound 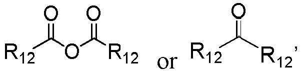 to react to obtain RM01-2;
(b) mixing compound RM01-2 with solvent b and catalyst b, adding a base, and then adding compounds R₂-R₁₄ to react to obtain compound RM01-3; and
(c) removing the R₁₃ group from compound RM01-3 to obtain compound RM01; 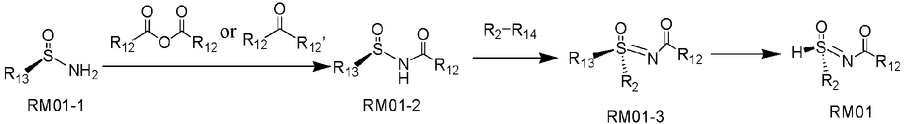 wherein
R₁₂' is halogen;
R₁₃ is C₁₋₁₀ alkyl, C₃₋₁₀ cycloalkyl, or C₆₋₁₈ aryl;
R₁₄ is a leaving group.

8. The preparation method according to claim 7, wherein the solvent a in step (a) is any one or more selected from the group consisting of: acetone, dioxane, tetrahydrofuran, tert-butanol and methylethyl alcohol;
the base in step (a) is a strong base or a weak base, which is selected from the group consisting of: sodium hydride, potassium hydride, potassium amide, sodium amide, sodium methoxide, sodium ethoxide, potassium ethoxide, sodium tert-butoxide, potassium tert-butoxide, butyl lithium, lithium diisopropylamide, benzyl lithium, Grignard reagent, DBU, potassium carbonate, sodium carbonate, potassium bicarbonate, sodium bicarbonate, triethylamine and pyridine, preferably sodium hydride, potassium tert-butoxide or potassium carbonate;
the reaction temperature in step (a) is 20-40°C;
the solvent b in step (b) is any one or more selected from the group consisting of: ethyl acetate, isopropanol, chloroform, methylethyl ketone, dioxane, pyridine and tetrahydrofuran, preferably dioxane;
the catalyst b in step (b) is a crown ether, preferably 18-crown ether-6 or 15-crown ether-5;
the base in step (b) is a strong base, preferably any one selected from the group consisting of: sodium hydride, potassium hydride, potassium amide, sodium amide, sodium methoxide, sodium ethoxide, potassium ethoxide, sodium tert-butoxide, potassium tert-butoxide, butyl lithium, lithium diisopropylamide, benzyl lithium, Grignard reagent and DBU, more preferably sodium hydride or potassium tert-butoxide; and
the reaction temperature in step (b) is 35-50°C.

9. The preparation method according to any one of claims 1 to 8, wherein R₄ is -CH₃;
A is C;
R₅ is selected from the group consisting of: H and -CH₃; 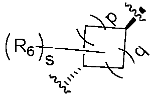 moiety is 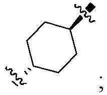
R₇ and R₈ are independently selected from the group consisting of: H and -CH₃;
R₁ and R₂ are independently selected from the group consisting of: C₁₋₆ alkyl, C₁₋₆ haloalkyl, C₃₋₆ cycloalkyl, C₃₋₆ halocycloalkyl, C₃₋₈ cycloalkyloxy, C₄₋₈ cycloalkylalkyl, C₂₋₆ alkoxyalkyl, C₆₋₁₀ aryl (e.g., phenyl), 4-10 membered heterocyclyl, C₆₋₁₀ aralkyl (e.g., benzyl, phenethyl) and heterocyclylalkyl; optionally, wherein the H on the carbon or nitrogen atom may be substituted by a group selected from the group consisting of: deuteration, hydroxyl, halogen, -CN, -CH₂-CN, -OCH₂F, -OCHF₂ and -OCF₃;
preferably, R₁ is selected from the group consisting of: -CH₃, -CH₂CH₃, -CH₂CH₂CH₃, 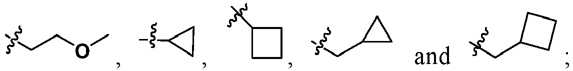
preferably, R₂ is selected from the group consisting of: -CH₃, -CH₂CH₃, -CH₂CH₂CH₃,

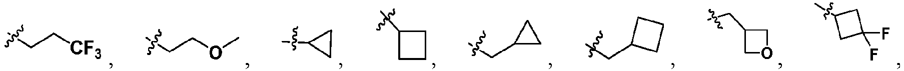

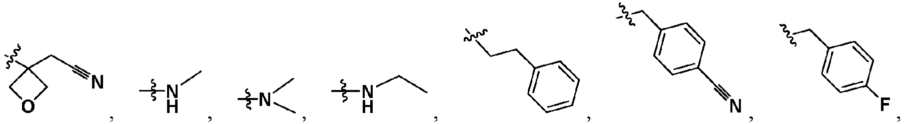

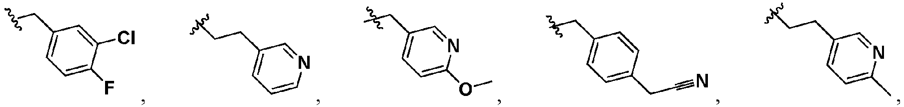

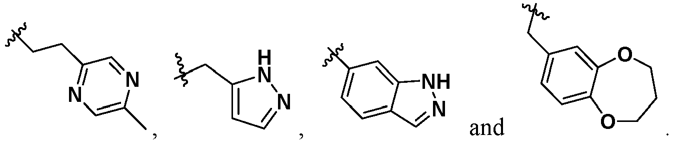

10. The preparation method according to claim 9, wherein the compound RM02 is 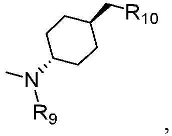 the preparation method further comprises the following reaction scheme: 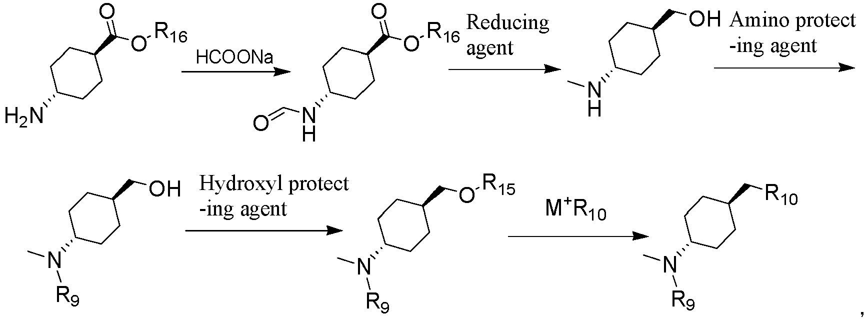 alternatively, 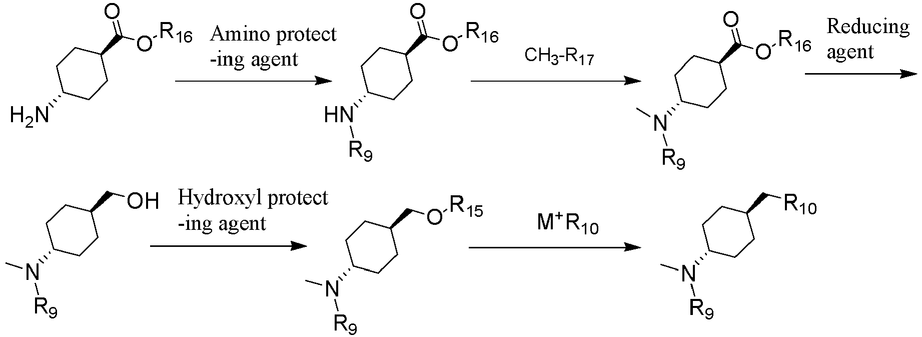 wherein
R₁₅ is a hydroxyl protecting group;
R₁₆ is H or alkyl;
R₁₇ is halogen;
M is an alkali metal.

11. A compound RM03 has the following structure: 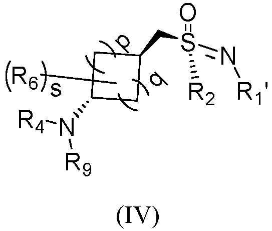 wherein
R₁' is an amino protecting group;
R₂ is selected from the group consisting of: halogen, -CN, -OCH₂F, -OCHF₂, -OCF₃, C₁₋₁₀ alkyl, C₃₋₁₀ cycloalkyl, -OC₀₋₁₀ alkyl, -N(C₀₋₁₀ alkyl)(C₀₋₁₀ alkyl), -O heterocycloalkyl, -N heterocycloalkyl, -S heterocycloalkyl, C₆₋₁₈ aryl, -N heterocyclic aryl, -S heterocyclic aryl or -O heterocyclic aryl, cycloalkylalkyl, aralkyl and heterocyclic alkyl, wherein the H on the carbon or nitrogen atom may be substituted by a group selected from: deuteration, hydroxyl, halogen, -CN, -OCH₂F, -OCHF₂, -OCF₃, C₁₋₆ straight chain alkyl, -N(C₀₋₁₀ alkyl)(C₀₋₁₀ alkyl), -OC₀₋₁₀ alkyl, C₃₋₁₀ cycloalkyl, -O heterocycloalkyl, -N heterocycloalkyl, -S heterocycloalkyl, C₆₋₁₈ aryl, -N heterocyclic aryl, -O heterocyclic aryl or -S heterocyclic aryl; and wherein the H on the C₆₋₁₈ aryl or heterocyclic aryl may be arbitrarily substituted by any one or more groups selected from: halogen, C₁₋₄ straight chain alkyl, -N(C₀₋₁₀ alkyl)SO₂(C₀₋₁₀ alkyl), -CON(C₀₋₁₀ alkyl)(C₀₋₁₀ alkyl), -N(C₀₋₁₀ alkyl)CO(C₀₋₁₀ alkyl), -N(C₀₋₁₀ alkyl)COO(C₀₋₁₀ alkyl), -OCON(C₀₋₁₀ alkyl)(C₀₋₁₀ alkyl), -CN, -OCH₂F, -OCHF₂, -OCF₃, -N(C₀₋₁₉ alkyl)(C₀₋₁₀ alkyl), -OC₀₋₁₀ alkyl, -N heteroaryl, -O heteroaryl or -S heteroaryl; or the adjacent carbon atoms on the C₆₋₁₈ aryl or heterocyclic aryl form a C₃₋₈ cycloalkyl, -O heterocycloalkyl, -N heterocycloalkyl, -S heterocycloalkyl, -N heterocyclic aryl, -O heterocyclic aryl or -S heterocyclic aryl;
R₄ is selected from the group consisting of: H, C₁₋₁₀ straight chain/branched alkyl, C₁₋₁₀ straight chain/branched alkenyl, C₁₋₁₀ straight chain/branched alkynyl, C₆₋₁₈ aryl, C₆₋₁₈ heterocyclic aryl, C₃₋₁₀ cycloalkyl, -OC₀₋₁₀ alkyl and -O heterocycloalkyl, wherein the H on the carbon atom may be substituted by a group selected from: deuteration, hydroxyl, halogen, -CN, -OCH₂F, -OCHF₂, -OCF₃, C₁₋₁₀ straight chain/branched alkyl, -N(C₀₋₁₀ alkyl)(C₀₋₁₀ alkyl), -OC₀₋₁₀ alkyl, C₃₋₁₀ cycloalkyl, -O heterocycloalkyl, -N heterocycloalkyl, -S heterocycloalkyl, C₆₋₁₈ aryl, -N heterocyclic aryl, -O heterocyclic aryl or -S heterocyclic aryl; and wherein the alkyl moiety of the group may be arbitrarily substituted by any one or more groups selected from: -SO₂, -SO₂N(C₀₋₁₀ alkyl)(C₀₋₁₀ alkyl), -N(C₀₋₁₀ alkyl)SO₂(C₀₋₁₀ alkyl), -CON(C₀₋₁₀ alkyl)(C₀₋₁₀ alkyl), -N(C₀₋₁₀ alkyl)CO(C₀₋₁₀ alkyl), -N(C₀₋₁₀ alkyl)COO(C₀₋₁₀ alkyl), -OCON(C₀₋₁₀ alkyl)(C₀₋₁₀ alkyl), halogen, -CN, -OCH₂F, -OCHF₂, -OCF₃, -N(C₀₋₁₀ alkyl)(C₀₋₁₀ alkyl), -OC₀₋₁₀ alkyl, C₆₋₁₈ aryl, -N heterocyclic aryl, -O heterocyclic aryl or -S heterocyclic aryl;
R₆ is a substituent group for H on one or more carbon atoms on a cycloalkyl group, each R₆ is independently selected from the group consisting of: C₁₋₆ alkyl and C₃₋₆ cycloalkyl; s is an integer from 0-8 (e.g., 0, 1, 2, 3, 4 or 5);
p is any integer from 0-4, q is any integer from 0-4, and p and q are not 0 at the same time; and
R₉ is an amino protecting group;
preferably, the compound has the following structure: 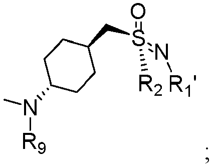
more preferably, R₁' is 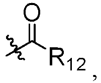 wherein R₁₂ is selected from the group consisting of: C₁₋₆ alkyl, C₁₋₆ haloalkyl and phenyl; further preferably, R₁' is

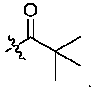

12. A compound RM06 has the following structure: 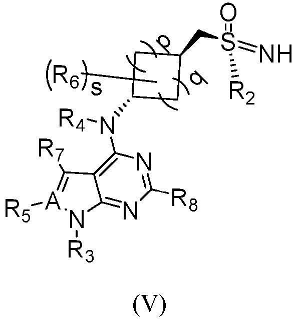 wherein
A is selected from: C or N; when A is N, R₅ is absent, and when A is C, R₅ is selected from the group consisting of: H, halogen, hydroxyl, cyano, substituted or unsubstituted alkyl, substituted or unsubstituted haloalkyl, substituted or unsubstituted alkoxy, substituted or unsubstituted hydroxyalkyl, substituted or unsubstituted cycloalkyl, substituted or unsubstituted heterocycloalkyl, substituted or unsubstituted amino, substituted or unsubstituted sulfonic acid group, and substituted or unsubstituted sulfonyl;
R₂ is selected from the group consisting of: halogen, -CN, -OCH₂F, -OCHF₂, -OCF₃, C₁₋₁₀ alkyl, C₃₋₁₀ cycloalkyl, -OC₀₋₁₀ alkyl, -N(C₀₋₁₀ alkyl)(C₀₋₁₀ alkyl), -O heterocycloalkyl, -N heterocycloalkyl, -S heterocycloalkyl, C₆₋₁₈ aryl, -N heterocyclic aryl, -S heterocyclic aryl or -O heterocyclic aryl, cycloalkylalkyl, aralkyl and heterocyclylalkyl, wherein the H on the carbon or nitrogen atom may be substituted by a group selected from: deuteration, hydroxyl, halogen, -CN, -OCH₂F, -OCHF₂, -OCF₃, C₁₋₆ straight chain alkyl, -N(C₀₋₁₀ alkyl)(C₀₋₁₀ alkyl), -OC₀₋₁₀ alkyl, C₃₋₁₀ cycloalkyl, -O heterocycloalkyl, -N heterocycloalkyl, -S heterocycloalkyl, C₆₋₁₈ aryl, -N heterocyclic aryl, -O heterocyclic aryl or -S heterocyclic aryl; and wherein the H on C₆₋₁₈ aryl or heterocyclic aryl may be arbitrarily substituted by any one or more groups selected from: halogen, C₁₋₄ straight chain alkyl, -N(C₀₋₁₀ alkyl)SO₂(C₀₋₁₀ alkyl), -CON(C₀₋₁₀ alkyl)(C₀₋₁₀ alkyl), -N(C₀₋₁₀ alkyl)CO(C₀₋₁₀ alkyl), -N(C₀₋₁₀ alkyl)COO(C₀₋₁₀ alkyl), -OCON(C₀₋₁₀ alkyl)(C₀₋₁₀ alkyl), -CN, -OCH₂F, -OCHF₂, -OCF₃, -N(C₀₋₁₀ alkyl)(C₀₋₁₀ alkyl), -OC₀₋₁₀ alkyl, -N heterocyclic aryl, -O heterocyclic aryl or -S heterocyclic aryl; or the adjacent carbon atoms on the C₆₋₁₈ aryl or heterocyclic aryl form a C₃₋₈ cycloalkyl, -O heterocycloalkyl, -N heterocycloalkyl, -S heterocycloalkyl, -N heterocyclic aryl, -O heterocyclic aryl or -S heterocyclic aryl;
R₃ is an amino protecting group;
R₄ is selected from the group consisting of: H, C₁₋₁₀ straight chain/branched alkyl, C₁₋₁₀ straight chain/branched alkenyl, C₁₋₁₀ straight chain/branched alkynyl, C₆₋₁₈ aryl, C₆₋₁₈ heterocyclic aryl, C₃₋₁₀ cycloalkyl, -OC₀₋₁₀ alkyl and -O heterocycloalkyl, wherein the H on the carbon atom may be substituted by a group selected from: deuteration, hydroxyl, halogen, -CN, -OCH₂F, -OCHF₂, -OCF₃, C₁₋₁₀ straight chain/branched alkyl, -N(C₀₋₁₀ alkyl)(C₀₋₁₀ alkyl), -OC₀₋₁₀ alkyl, C₃₋₁₀ cycloalkyl, -O heterocycloalkyl, -N heterocycloalkyl, -S heterocycloalkyl, C₆₋₁₈ aryl, -N heterocyclic aryl, -O heterocyclic aryl or -S heterocyclic aryl; and wherein the alkyl moiety of the group may be substituted by any one or more groups selected from: -SO₂, -SO₂N(C₀₋₁₀ alkyl)(C₀₋₁₀ alkyl), -N(C₀₋₁₀ alkyl)SO₂(C₀₋₁₀ alkyl), -CON(C₀₋₁₀ alkyl)(C₀₋₁₀ alkyl), -N(C₀₋₁₀ alkyl)CO(C₀₋₁₀ alkyl), -N(C₀₋₁₀ alkyl)COO(C₀₋₁₀ alkyl), -OCON(C₀₋₁₀ alkyl)(C₀₋₁₀ alkyl), halogen, -CN, -OCH₂F, -OCHF₂, -OCF₃, -N(C₀₋₁₀ alkyl)(C₀₋₁₀ alkyl), -OC₀₋₁₀ alkyl, C₆₋₁₈ aryl, -N heterocyclic aryl, -O heterocyclic aryl or -S heterocyclic aryl;
R₆ is a substituent group for H on one or more carbon atoms on a cycloalkyl group, each R₆ is independently selected from the group consisting of: C₁₋₆ alkyl and C₃₋₆ cycloalkyl; s is an integer from 0-8 (e.g., 0, 1, 2, 3, 4 or 5);
p is any integer from 0-4, q is any integer from 0-4, and p and q are not 0 at the same time; and
R₇ and R₈ are independently selected from the group consisting of: H, halogen, hydroxyl, cyano, substituted or unsubstituted alkyl, substituted or unsubstituted alkenyl, substituted or unsubstituted alkynyl, substituted or unsubstituted haloalkyl, substituted or unsubstituted alkoxy, substituted or unsubstituted hydroxyalkyl, substituted or unsubstituted cycloalkyl, substituted or unsubstituted non-heterocyclic aryl, substituted or unsubstituted heterocyclic aryl, substituted or unsubstituted heterocycloalkyl, substituted or unsubstituted amino, substituted or unsubstituted sulfonic acid group, and substituted or unsubstituted sulfonyl;
preferably, the compound has the following structure:

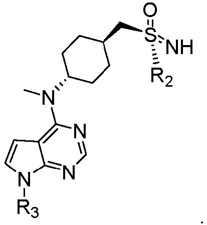

13. The compound according to claim 11 or 12, wherein R₂ is selected from the group consisting of: C₁₋₆ alkyl, C₁₋₆ haloalkyl, C₃₋₆ cycloalkyl, C₃₋₆ halocycloalkyl, C₃₋₈ cycloalkyloxy, C₄₋₈ cycloalkylalkyl, C₂₋₆ alkoxyalkyl, C₆₋₁₀ aryl (e.g., phenyl), 4-10 membered heterocyclyl, C₆₋₁₀ aralkyl (e.g., benzyl, phenethyl) and heterocyclylalkyl; optionally, the H on the carbon or nitrogen atom may be substituted by a group selected from the group consisting of: deuteration, hydroxyl, halogen, -CN, -CH₂-CN, -OCH₂F, -OCHF₂, and -OCF₃;
preferably, R₂ is selected from the group consisting of: -CH₃, -CH₂CH₃, -CH₂CH₂CH₃,

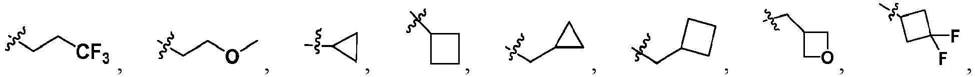

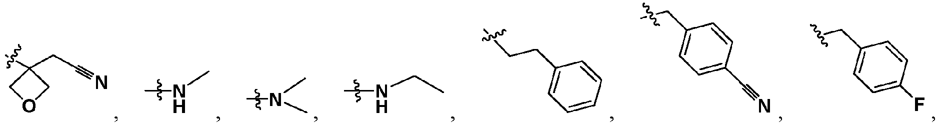

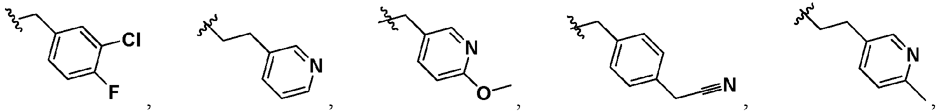

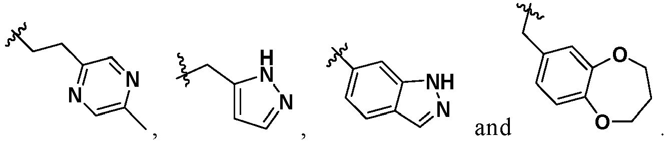

14. A preparation method of the compound according to claim 12 or 13, comprising the steps of:
(A) mixing compound RM03 with an acid to react at 50-65°C;
(B) adjusting the pH to weak alkalinity; and
(C) adding a base, solvent 3, and compound RM05 to react at 55-70°C;

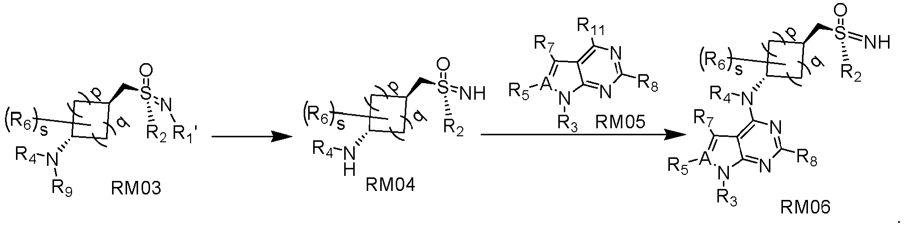

15. The preparation method according to claim 14, wherein step (A) comprises: mixing compound RM03 with an acid, stirring at 20-30°C, and then stirring at 50-65°C; and/or,
step (B) comprises: adjusting the pH to weak alkalinity, and stirring at 20-30°C.
